(19) Europäisches Patentamt
European Patent Office
Office européen des brevets

(11) EP 3 210 528 A1

(12) EUROPEAN PATENT APPLICATION

(43) Date of publication:
30.08.2017 Bulletin 2017/35

(51) Int Cl.:
A61B 5/00 (2006.01)    A61B 5/024 (2006.01)
A61B 5/11 (2006.01)

(21) Application number: 17153497.7

(22) Date of filing: 27.01.2017

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 25.02.2016   JP 2016034764

(71) Applicant: FUJITSU LIMITED
Kawasaki-shi Kanagawa 211-8588 (JP)

(72) Inventor: Yamaji, Takayuki
Kawasaki-shi
Kanagawa 211-8588 (JP)

(74) Representative: Hoffmann Eitle
Patent- und Rechtsanwälte PartmbB
Arabellastraße 30
81925 München (DE)

(54) SENSOR INFORMATION PROCESSING APPARATUS

(57) An extended wavelength defined as a length of a signal waveform corresponding to a locus traced by a detected signal of an inertial sensor (22) in a time domain per unit time is calculated, and an activity state of an observed person observed by the inertial sensor (22) is determined based on the extended wavelength.

FIG. 1

1

**Description**

FIELD

The embodiment (s) discussed herein is related to a sensor information processing apparatus.

BACKGROUND

There is a technique of determining a state (e.g. whether a state is a dynamic state or a static state) related to a behavior or a motion of a user to which an acceleration sensor is attached, based on a measured value of the acceleration sensor (e.g. a level of an acceleration).

By, for example, comparing measured values of the triaxial acceleration sensor with a threshold value (which may be referred to as a "reference value") per detection axis, or applying an affine transformation matrix to the measured values of the triaxial acceleration sensor, and converting the measured values into data of a reference coordinate system, a state related to a user's behavior or motion is determined. In addition, the state related to the user's behavior or motion may be referred to as an "activity state" for descriptive purposes.

(RELATED ART DOCUMENTS LIST)

Patent Document 1 JP1-115344 A
Patent Document 2 JP2011-115459 A
Patent Document 3 JP2006-68300 A
Patent Document 4 JP2004-81632 A
Patent Document 5 JP2014-94043 A

However, according to each one of the above techniques, the measured values are analyzed based on an acceleration in a gravity direction. Therefore, when, for example, the acceleration sensor is not attached to the user in a desired direction, the measured values mismatch with the reference value or the reference coordinate.

As a result, a difference in an angle to attach the acceleration sensor to the user changes an analysis result and lowers an accuracy to determine a user's behavior. When, for example, the acceleration sensor is put in a diagonally inclined state in a pocket of the clothes the user puts on, it is difficult to correctly determine the user's behavior.

If the reference value or the reference coordinate used to analyze a measured value is reset according to the angle to attach the acceleration sensor to the user, while it is possible to reduce erroneous determination, convenience lowers.

SUMMARY

According to one aspect, one of objects of the technique discussed herein is to improve a determination accuracy of an activity state of an observed person based on a detected signal of an inertial sensor.

In one aspect, a sensor information processing apparatus may include a receiver and a processor. The processor may calculate an extended wavelength defined as a length of a signal waveform corresponding to a locus traced by the detected signal in a time domain per unit time, and may determine an activity state of an observed person observed by the inertial sensor based on the extended wavelength.

Further, according to one aspect, a sensor unit may include an inertial sensor and a processor. The processor may calculate an extended wavelength defined as a length of a signal waveform corresponding to a locus traced by the detected signal in a time domain per unit time, and may determine an activity state of an observed person observed by the inertial sensor based on the extended wavelength.

According to one aspect, it is possible to improve a determination accuracy of an activity state of an observed person based on a detected signal of the inertial sensor.

BRIEF DESCRIPTION OF DRAWINGS

FIG. 1 is a block diagram illustrating an example of a sensor system according to an embodiment;
FIGS. 2 and 3 are block diagrams illustrating configuration examples of a vital sensor according to an embodiment;

FIG. 4 is a block diagram illustrating a configuration example of an information processing apparatus according to an embodiment;

FIG. 5A is a diagram schematically illustrating detection axes of an inertial sensor illustrated in FIG. 2;

FIG. 5B is a diagram schematically illustrating a state where the inertial sensor illustrated in FIG. 5A is attached of a human body;

FIG. 6 is a flowchart illustrating an operation example of a sensor system according to one embodiment;

FIG. 7 is a diagram schematically illustrating a concept of an extended wavelength according to one embodiment;

FIG. 8 is a diagram illustrating a calculation example of the extended wavelength according to one embodiment;

FIGS. 9A to 9C are diagrams illustrating examples of an extended wavelength calculation process according to one embodiment;

FIG. 10 is a diagram illustrating another calculation example of an extended wavelength according to one embodiment;

FIG. 11 is a diagram illustrating an example of an activity of an observed person according to one embodiment;

FIG. 12 is a diagram illustrating an example of a 1-second extended wavelength (Ly) on the detection axis (Y) of the inertial sensor according to one embodiment;

FIG. 13 is a diagram illustrating an example of a 15-second extended wavelength (Ly) on the detection axis (Y) of the inertial sensor according to one embodiment;

FIG. 14 is a flowchart illustrating a first example of an activity state determination process illustrated in FIG. 6;

FIG. 15 is a flowchart illustrating a second example of an activity state determination process illustrated in FIG. 6;

FIG. 16 is a diagram illustrating an example of an activity of an observed person according to one embodiment;

FIG. 17A is a diagram illustrating an example of the 1-second extended wavelength (Ly) on the detection axis (Y) of the inertial sensor according to one embodiment;

FIG. 17B is a diagram illustrating an example of a 1-second extended wavelength (Lz) on a detection axis (Z) of the inertial sensor according to one embodiment;

FIG. 18A is a diagram illustrating an example of the 15-second extended wavelength (Lz) on the detection axis (Y) of the inertial sensor according to one embodiment;

FIG. 18B is a diagram illustrating an example of the 15-second extended wavelength (Lz) on the detection axis (Z) of the inertial sensor according to one embodiment;

FIG. 19 is a flowchart illustrating an example of a heartbeat detection algorithm determination process illustrated in FIG. 6;

FIG. 20 is a flowchart illustrating an example of a first heartbeat detection process example illustrated in FIG. 19;

FIG. 21 is a flowchart illustrating a first example of a second heartbeat detection process example illustrated in FIG. 19;

FIG. 22 is a flowchart illustrating a first example of a heart rate estimation process illustrated in FIG. 21;

FIG. 23 is a diagram illustrating an example of a relation between a walking cadence and a heart rate according to one embodiment;

FIG. 24 is a flowchart illustrating a second example of the heart rate estimation process illustrated in FIG. 21;

FIG. 25 is a diagram illustrating an example of a relation between a walking cadence and a walking speed according to one embodiment;

FIG. 26 is a diagram illustrating an example of a relation between a walking speed and an exercise intensity (METs value) according to one embodiment;

FIG. 27 is a flowchart illustrating a third example of the heart rate estimation process illustrated in FIG. 21;

FIG. 28A is a diagram illustrating an example of a relation between an exercise intensity and an extended wavelength (Lx) on a detection axis (X) of the inertial sensor according to one embodiment;

FIG. 28B is a diagram illustrating an example of a relation between an exercise intensity and the extended wavelength (Ly) on the detection axis (Y) of the inertial sensor according to one embodiment;

FIG. 29 is a flowchart illustrating a second example of a second heartbeat detection process example illustrated in FIG. 19;

FIG. 30 is a flowchart illustrating an example of a third heartbeat detection process example illustrated in FIG. 19;

FIG. 31 is a diagram illustrating an example of a heart rate measurement result according to one embodiment;

FIG. 32 is a diagram illustrating a setting example of a BPF according to one embodiment;

FIG. 33 is a diagram illustrating an example of a passband of the BPF according to one embodiment;

FIG. 34 is a diagram illustrating a setting example of a bandwidth of a BPF according to one embodiment;

FIGS. 35 and 36 are diagrams illustrating examples of distributions of the heart rate according to an embodiment;

FIGS. 37 and 38 are diagrams illustrating examples of a statistical process of the heart rate according to an embodiment;

FIGS. 39 and 40 are diagrams illustrating setting examples of a bandwidth of a BPF according to one embodiment; and

FIG. 41 is a diagram illustrating an example of bandwidth information of the BPF according to an embodiment.

DESCRIPTION OF EMBODIMENTS

Hereinafter, an exemplary embodiment(s) will be described with reference to the drawings. However, the embodiment(s) described below is merely an example and not intended to exclude an application of various modifications or techniques which are not explicitly described below. Further, various exemplary aspects described below may be appropriately combined and carried out. Elements or components assigned the same reference numeral in the drawings used for the following embodiment(s) will represent identical or similar elements or components unless otherwise specified.

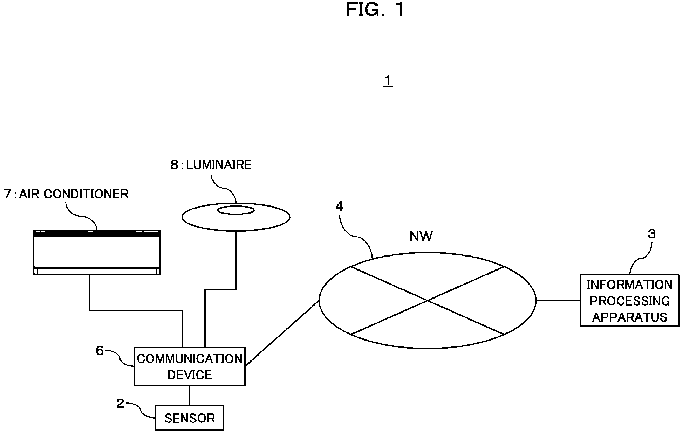

FIG. 1 is a block diagram illustrating an example of a sensor system according to an embodiment. A sensor system 1 illustrated in FIG. 1 may illustratively include a sensor 2, an information processing apparatus 3 and a network (NW) 4.

The sensor 2 may illustratively be connected to the network 4 through a communication device 6, and may be available to communicate with the information processing apparatus 3 through the network 4.

The sensor 2 is available to sense information of a biological object (hereinafter, may be referred to as "vital information"). Hence, the "sensor 2" may be referred to as the "vital sensor 2" for descriptive purposes. Further, "sensing" may be referred to as "detection" or "measurement".

A non-restrictive example of the "vital information" is information indicative of a heartbeat of a biological object. Since a blood vessel of the biological object pulsates according to a heartbeat, the information indicative of the heartbeat may be considered as being equivalent to information indicative of a pulsebeat.

The heartbeat or the pulsebeat of the biological object can be illustratively acquired as a change in an electromagnetic wave, a pressure or sound corresponding to a heartbeat.

For example, when a blood vessel of a finger or an earlobe is irradiated with light such as infrared rays, the reflected light would be change periodically according to a rhythmical change in a bloodstream and light absorption characteristics. Hence, a heartbeat or a pulsebeat can be optically measured as a fluctuation of reflected light according to a change in a bloodstream.

Alternatively, when a biological object is irradiated with a radio wave such as a microwave, a rhythmical motion would occur in a surface of a biological object (e.g. skin) according to a heartbeat. Therefore, a distance between the skin and a radio wave transmission source changes according to the motion, and changes due to a Doppler effect would occur in a reflected wave. Hence, it is also possible to measure a heartbeat or a pulsebeat as a fluctuation caused by the Doppler effect of the reflected wave irradiated on the biological object.

Further, when a heart is contracted and relaxed rhythmically, a pressure of a blood vessel (hereinafter, may be referred to as a "blood pressure") would also be fluctuated. Therefore, it is also possible to measure a heartbeat or a pulsebeat as a rhythmical fluctuation of a blood pressure by using a pressure sensor or a piezoelectric sensor.

Furthermore, it is also possible to measure a heartbeat as a change in an electric potential or a change in sound of a heart muscle corresponding to a heart pulse by using an electrocardiograph or a phonocardiograph.

A sensor available to measure a heartbeat using any one of the above measurement schemes may be referred to as a "heartbeat sensor" or a "heartbeat meter". As described above, since the information indicative of a "heartbeat" may be considered as being equivalent to the information indicative of a "pulsebeat" in some cases, the "heartbeat sensor" or the "heartbeat meter" may be referred to as a "pulsebeat sensor" or a "pulsebeat meter".

In the present embodiment, the vital sensor 2 may include a "wireless sensor" which can measure a heartbeat by using the Doppler effect as an example of a heartbeat sensor for descriptive purposes. In addition, the "wireless sensor" maybe referred to as a "microwave sensor", a "RF (Radio Frequency) sensor", a "UWB (Ultra Wide Band)" or a "Doppler sensor" in some cases.

The vital sensor 2 may be illustratively attached in contact with a skin of a human body or may be attached to clothes of the human body. The vital sensor 2 does not need to be attached to a human body by way of strictly fixing (may be referred to as "restraining"). It may be allowed to occur a relative motion between the vital sensor 2 and a human body according to a mismatch between motions of the clothes and a human body surface.

A relative motion (which may be referred to as an "asynchronous motion") may be permitted to be made between the vital sensor 2 and a human body according to a mismatch between motions of the clothes and a human body surface.

For example, the vital sensor 2 may be attached to a human body such that the vital sensor 2 is allowed to be moved in one of three-dimensional directions relative to a human body.

Illustratively, the vital sensor 2 may be put in a pocket of clothes such as a breast pocket of a jacket or may be attached on the clothes by using an attachment tool such as a harness.

Next, the communication device 6 illustrated in FIG. 1 is available to transmit a sensing result (e.g. the information indicative of the heartbeat) of the vital sensor 2 to the information processing apparatus 3 through the network 4, for example. Hence, the communication device 6 may be connected with the network 4 using a wired cable or radio.

In other words, the communication device 6 may be provided with a communication interface (IF) which supports one or both of wireless and wired communications. Illustratively, a communication scheme based on the LTE (Long Term Evolution) or the LTE-Advanced of 3GPP (3rd Generation Partnership Project) is applicable to the wireless communication of the communication device 6.

Further, satellite communications may be applied to the wireless communication of the communication device 6. When the satellite communication is applied, the communication device 6 may be able to communicate with the information processing apparatus 3 through a communication satellite without being routed through the network 4.

The sensing result of the vital sensor 2 may include not only vital information but also information indicative of a result of an arithmetic operation or determination, which is obtained based on the vital information. The sensing result maybe referred to as "sensor information" or "sensor data" for descriptive purposes.

The communication device 6 may be externally attached to the vital sensor 2 as illustrated in FIG. 1, or may be built in the vital sensor 2. The communication device 6 externally attached to the vital sensor 2 may be, for example, a device carried by a person attached with the vital sensor 2. The person who the vital sensor 2 is attached to may be referred to as a "user", a "subject" or an "observed person" for descriptive purposes.

The communication device 6 carried by the user may be illustratively a mobile telephone (which may include a smartphone), a notebook PC or a tablet PC. The "PC" is an abbreviation of a "personal computer".

A wired connection or a wireless connection may be applied to a connection between the vital sensor 2 and the communication device 6. In other words, the vital sensor 2 may be provided with a communication IF which supports one or both of wireless and wired communications. The "WiFi (Wireless Fidelity)" (registered trademark) or "Bluetooth" (registered trademark) may also be applied to the wireless connection.

The communication device 6 externally attached to the vital sensor 2 may be a router or a network switch. As illustrated in FIG. 1, the communication device 6 may be communicably connected with an air conditioner 7 and an luminaire 8 so that the air conditioner 7 and the luminaire 8 are able to communicate with the information processing apparatus 3 through the network 4.

The network 4 may be illustratively a WAN (Wide Area Network), a LAN (Local Area Network) or the Internet. Further, the network 4 may include a wireless access network. The wireless access network may be compliant with the above-described LTE or LTE-Advanced.

The information processing apparatus 3 receives the sensor information of the vital sensor 2 through the network 4 (or may be through the communication satellite), and processes the received sensor information. Hence, the information processing apparatus 3 may be referred to as the "sensor information processing apparatus 3" for descriptive purposes.

Processing the sensor information may include storing and managing the sensor information, and estimating a heart rate and an exercise intensity of the user based on the sensor information. Hence, the information processing apparatus 3 can monitor an activity state of the user, for example. In other words, the sensor system 1 can provide a user "monitoring (or watching) function".

Managing the sensor information may include compiling the sensor information in a database (DB). The data compiled in the DB may be referred to as "cloud data" or "big data".

The information processing apparatus 3 may be illustratively realized by one or a plurality of servers. In other words, the sensor information obtained by the vital sensor 2 may be processed or managed by a single server or may be distributedly processed or managed by a plurality of servers in the information processing apparatus 3. The server may correspond to a cloud server provided in a cloud data sensor, for example.

The information processing apparatus 3 may be communicably connected with the vital sensor 2 without being routed through the network 4. For example, the information processing apparatus 3 may be available to directly receive the sensor information from the vital sensor 2 through a wired cable or by radio.

(Configuration example of vital sensor 2)

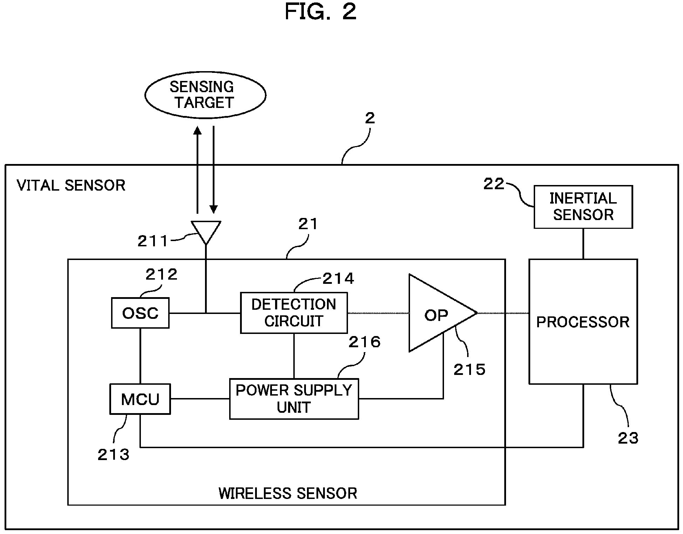

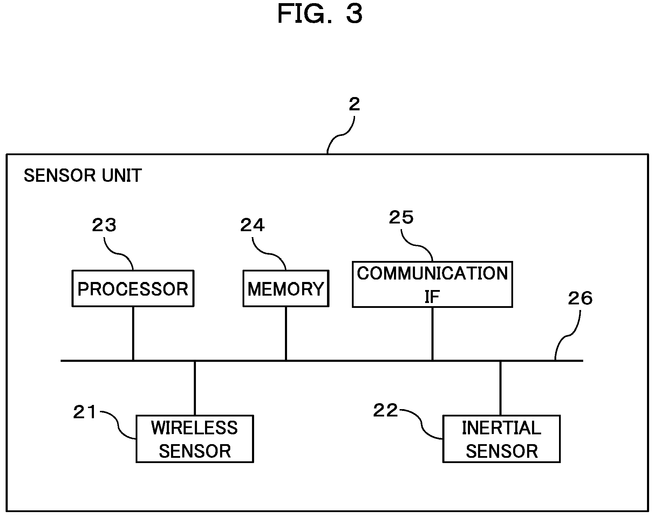

Next, the configuration example of the vital sensor 2 will be described with reference to FIGS. 2 and 3. As illustrated in FIGS. 2 and 3, the vital sensor 2 may illustratively include a wireless sensor 21, an inertial sensor 22, a processor 23, a memory 24 and a communication IF 25. The wireless sensor 21 may be optional.

The vital sensor 2 may be referred to as the sensor unit 2. Hereinafter, the vital sensor 2 or the sensor unit 2 will be simply referred to as the "sensor 2" for descriptive purposes.

As illustrated in FIG. 3, the wireless sensor 21, the inertial sensor 22, the processor 23, the memory 24 and the communication IF 25 may be illustratively connected to a bus 26 to communicate with each other through the processor 23.

The wireless sensor 21 is an example of the heartbeat sensor and may be illustratively a Doppler sensor. The wireless sensor 21 may perform phase detection on a radio wave transmitted to a space and a reflected wave of the transmitted radio wave, and generate a beat signal. The beat signal may be supplied as an output signal of the wireless sensor 21 to the processor 23.

As illustrated in FIG. 2, the wireless sensor 21 may include, for example, an antenna 211, a local oscillator (OSC) 212, a MCU (Micro Control Unit) 213, a detection circuit 214, an operational amplifier (OP) 215 and a power supply unit (or a power supply circuit) 216.

The antenna 211 transmits to the space a radio wave having an oscillation frequency generated by the OSC 212, and receives a reflected wave of the transmitted radio wave reflected by the user positioned in the space. In an example of FIG. 2, the antenna 211 is shared by a transmission and a reception but a transmission antenna and a reception antenna may be provided individually.

The OSC 212 illustratively oscillates in response to a control of the MCU 213 to output a signal with a predetermined frequency (which may be referred to as a "local signal" for descriptive purposes). The local signal is transmitted as a transmission radio wave from the antenna 211, and is inputted to the detection circuit 214.

The oscillation frequency of the OSC 212 (in other words, a frequency of a radio wave transmitted by the wireless sensor 21) may be illustratively a frequency in a microwave band. The microwave band may be illustratively a 2.4 GHz band or a 24 GHz band.

These frequency bands are examples whose indoor use is authorized by the Radio Act in Japan. Frequency bands which are not regulated by the Radio Act may be used for a transmission radio wave of the wireless sensor 21.

The MCU 213 illustratively controls an oscillating operation of the OSC 212 in response to a control of the processor 23.

The detection circuit 214 illustratively performs the phase detection on the reflected wave received by the antenna 211 and the local signal (in other words, the transmission radio wave) from the OSC 212 to output the beat signal.

The detection circuit 214 may be replaced with a mixer which mixes a transmission radio wave and a reflected wave. The mixing performed by the mixer may be considered as being equivalent to the phase detection.

In this regard, a change in an amplitude and a change in a frequency corresponding to a heartbeat of the user occur in the beat signal obtained by the detection circuit 214 due to the Doppler effect. In other words, the beat signal includes information indicative of the heartbeat of the user.

The operational amplifier 215 illustratively amplifies the beat signal outputted from the detection circuit 214. The amplified beat signal is inputted to the processor 23. An amplification factor of the operational amplifier 215 may be illustratively variable. The variable amplification factor may be illustratively controlled by the processor 23.

The power supply unit 216 illustratively supplies drive power to the MCU 213, the detection circuit 214 and the operational amplifier 215.

The inertial sensor 22 may illustratively detect a motion of the sensor unit 2. The inertial sensor 22 may be an acceleration sensor or a gyroscope. Any one of piezoelectric type and capacitive type sensors may be illustratively applied to the acceleration sensor. Any one of a spin rotor (flywheel) type, an optical type and a vibrating structure type sensors may be applied to the gyroscope.

The inertial sensor 22 may include one or more of detection axes. A gravity component in a direction along one of the detection axes may be detected as an "acceleration" component, for example. A detected signal of the inertial sensor 22 may be inputted to the processor 23.

The processor 23 is an example of an arithmetic processing apparatus with a capability of arithmetic processing. The arithmetic processing apparatus may be referred to as an arithmetic processing device or an arithmetic processing circuit. An integrated circuit (IC) such as an MPU (Micro Processing Unit) or a DSP (Digital Signal Processor) may be illustratively applied to the processor 23 which is an example of the arithmetic processing apparatus. The "processor" may be referred to as a "processing unit", a "controller" or a "computer".

The processor 23 is available to detect the heartbeat of the user based on the detected signal of the wireless sensor 21. A filter may be illustratively applied to the detected signal of the wireless sensor 21 to detect a heartbeat derived signal component (which may be referred to as a "heartbeat component" or a "heartbeat signal") from the detected signal of the wireless sensor 21.

A bandpass filter (BPF) may be applied to the filter of a non-restrictive example. The processor 23 may determine a state related to a sleep of the user based on the detected heartbeat.

The detected signal of the inertial sensor 22 may be used to control filter characteristics (illustratively, a pass center frequency and passband width) of the above BPF. Controlling the filter characteristics may be considered as controlling a target frequency band to be processed in the detected signal of the wireless sensor 21. An example of a control of the filter characteristics will be described below.

The "pass center frequency" and the "passband width" of the BPF may be referred to simply as a "center frequency" and a "bandwidth", respectively.

The detected signal of the wireless sensor 21 and the detected signal of the inertial sensor 22 may be both referred to as a "detected value" or an "output value". For descriptive purposes, the detected value of the wireless sensor 21 may be referred to as a "wireless sensor value" and the detected value of the inertial sensor 22 may be referred to as an "inertial sensor value".

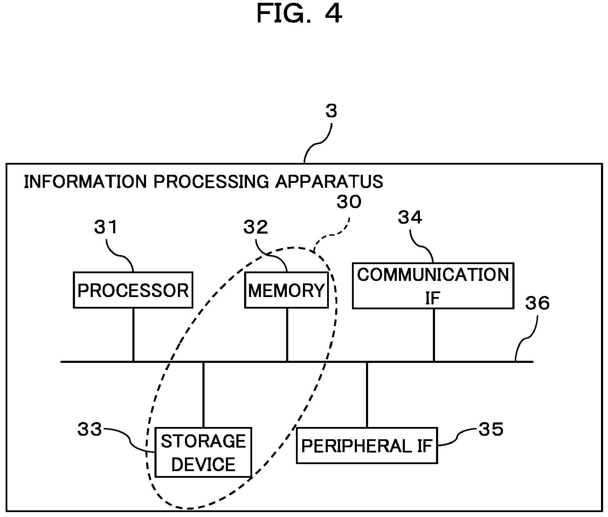

Further, the above-described detection of the heartbeat and control of the filter characteristics may be performed by a processor 31 of the information processing apparatus 3 (described below with reference to FIG. 4) instead of the processor 23 of the sensor unit 2.

Next, in FIG. 3, the memory 24 is an example of a storage unit or a storage medium provided in the sensor unit 2, and may be a RAM (Random Access Memory) or a flash memory.

A program and data read and used by the processor 23 to operate may be stored in the memory 24. The "program" may be referred to as a "software" or an "application". The "data" may include data generated according to operations of the processor 23.

The communication IF 25 is an example of a communication unit of the sensor unit 2, and is illustratively connected with the communication device 6 (see FIG. 1) and enables communication with the information processing apparatus 3 through the network 4.

For example, the communication IF 25 may transmit the detected signals of the wireless sensor 21 and the inertial sensor 22, and transmit information obtained based on one or both of the detected signals to the information processing apparatus 3.

In other words, the sensor information transmitted from the vital sensor 2 to the information processing apparatus 3 may include measured values of the wireless sensor 21 and the inertial sensor 22, or may include information obtained based on one or both of the measured values.

The communication IF 25 may be connected with the information processing apparatus 3 to directly communicate with the information processing apparatus 3 without being routed through the communication device 6 and/or the network 4.

(Configuration example of information processing apparatus 3)

Next, the configuration example of the information processing apparatus 3 illustrated in FIG. 1 will be described with reference to FIG. 4. As illustrated in FIG. 4, the information processing apparatus 3 may illustratively include the processor 31, a memory 32, a storage device 33, a communication interface (IF) 34 and a peripheral IF 35.

The processor 31, the memory 32, the storage device 33, the communication IF 34 and the peripheral IF 35 may be illustratively connected to a bus 36 to communicate with each other through the processor 31.

The processor 31 is an example of an arithmetic processing apparatus which has a capability of arithmetic processing. The arithmetic processing apparatus maybe referred to as an arithmetic processing device or an arithmetic processing circuit. An IC such as a CPU or a MPU or a DSP may be illustratively applied to the processor 31 which is an example of the arithmetic processing apparatus. The "processor" may be referred to as a "processing unit", a "controller" or a "computer".

The processor 31 illustratively controls an entire operation of the information processing apparatus 3. The control performed by the processor 31 may include a control for communication performed through the network 4. By controlling the communication, the air conditioner 7 and the luminaire 8 may be remotely controlled through the network 4, for example.

Illustratively, the processor 31 may detect the heartbeat or control the filter characteristics as described above based on the sensor information of the vital sensor 2, which is received by the communication IF 34.

Further, the processor 31 may illustratively generate a control signal to control a spatial environment in which the user of the vital sensor 2 is positioned, such as a control signal to control operations of the air conditioner 7 and the luminaire 8.

The control signal may be illustratively generated based on a heart rate of the user detected based on the sensor information obtained from the vital sensor 2 and a state related to a sleep of the user estimated or determined based on the heart rate.

The control signal generated by the processor 31 may be illustratively transmitted to the air conditioner 7 and the luminaire 8 through the communication IF 34.

The memory 32 is an example of a storage medium, and may be a RAM or a flash memory. A program and data read and used by the processor 31 to operate may be stored in the memory 32.

The storage device 33 may store various pieces of data and programs. A hard disk drive (HDD), a solid state drive (SSD) or a flash memory may be applied to the storage device 33.

The data stored in the storage device 33 may illustratively include the sensor information of the sensor 2 received by the communication IF 34, the heart rate detected based on the sensor information, and the state related to the sleep of the user estimated or determined based on the heart rate.

The data stored in the storage device 33 may be optionally compiled in a database (DB). The data compiled in the DB may be referred to as "cloud data" or "big data". The storage device 33 and the memory 32 may be collectively referred to as a "storage unit 30" of the information processing apparatus 3.

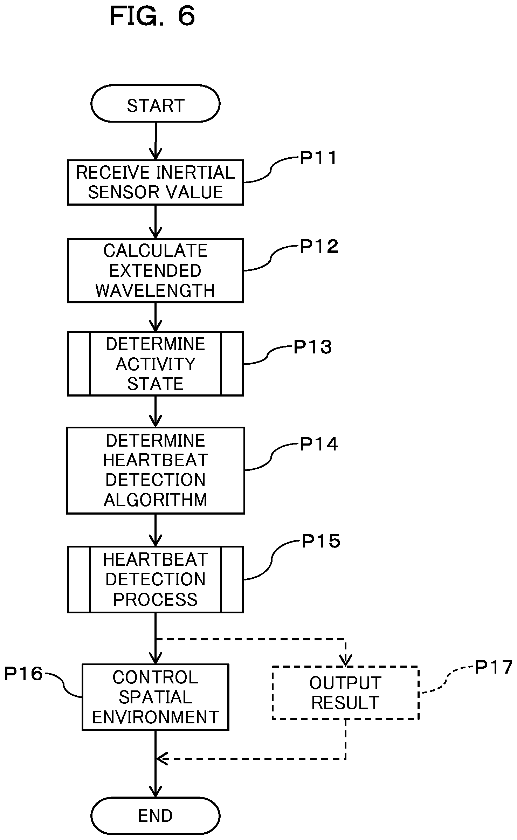

The programs stored in the storage unit 30 may include programs for executing one or more processes described below with reference to FIGS. 6, 14, 15, 19 to 21, 29 and 30.

The program to execute the processes described below with reference to FIGS. 6, 14, 15, 19-21, 29 and 30 may be referred to as a "sensor information processing program" for descriptive purposes.

All or part of program codes configuring a program may be stored in the storage unit or may be described as a part of an operating system (OS).

The program and the data may be recorded in a computer-readable non-transitory recording medium to be provided. Examples of the recording medium include flexible disks, CD-ROMs, CD-Rs, CD-RWs, MOs, DVDs, Blu-ray disks and portable hard disks. Further, a semiconductor memory such as a USB (Universal Serial Bus) memory is an example of a recording medium.

Alternatively, the program and the data may be provided (or downloaded) from the server to the information processing apparatus 3 through the network 4. For example, the program and the data may be provided to the information processing apparatus 3 through the communication IF 34. Further, the program and the data may be inputted to the information processing apparatus 3 from an input device described below which is connected to the peripheral IF 35.

The communication IF 34 is an example of a communication unit of the information processing apparatus 3, and is illustratively connected to the network 4 to enable communication via the network 4.

Focusing on a reception process, the communication IF 34 is an example of a receiver (which may be referred to as an "obtaining unit") which receives information transmitted from the vital sensor 2 to the information processing apparatus 3.

Meanwhile, focusing on a transmission process, for example, the communication IF 34 is an example of a transmitter which transmits the control signal generated by the processor 31 to the vital sensor 2, the air conditioner 7 and the luminaire 8. An Ethernet (registered trademark) card may be illustratively applied to the communication IF 34.

In addition, the communication IF 34 may be connected with the communication IF 25 of the vital sensor 2 without the network 4 to enable direct communication with the vital sensor 2.

The peripheral IF 35 is illustratively an interface which connects peripheral devices to the information processing apparatus 3.

The peripheral devices may include an input device which inputs information to the information processing apparatus 3, and an output device which outputs information generated by the information processing apparatus 3.

The input device may include a keyboard, a mouse and a touch panel. The output device may include a display and a printer.


(Attachment example of inertial sensor 22)

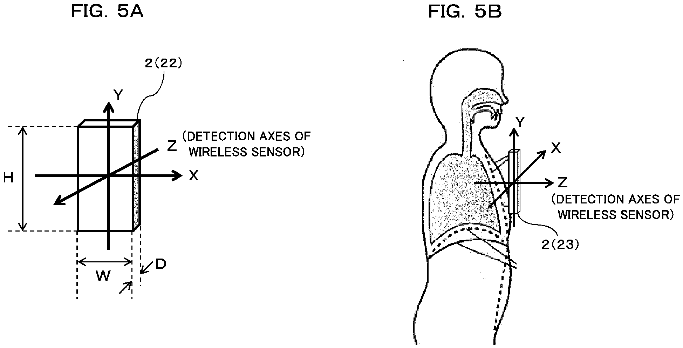

FIG. 5A illustrates an example of detection axes of the inertial sensor 22, and FIG. 5B is a side view schematically illustrating that the inertial sensor 22 (in other words, the "vital sensor 2") is attached to a human body.

The inertial sensor 22 illustrated in FIG. 5A is a triaxial inertial sensor including the three detection axes (X, Y and Z) orthogonal to each other. Illustratively, the detection axis Y is an example of a first detection axis, the detection axis Z is an example of a second detection axis, and the detection axis X is an example of a third detection axis.

The inertial sensor 22 illustratively has a size of a width W [cm] $\times$ a height H [cm] $\times$ a thickness D [cm]. This size of a non-restrictive example is W = 3 [cm], H = 5 [cm] and D = 1 [cm].

As a non-restrictive example, as illustrated in FIG. 5A, the detection axis X may be set in a width (W) direction of the inertial sensor 22, the detection axis Y may be set in a height (H) direction and the detection axis Z may be set in a thickness (D) direction.

Further, as illustrated in FIG. 5B, the inertial sensor 22 may be attached to a human body such that a plane formed by the detection axes X and Y has a positional relation parallel to a human body surface.

When, for example, the vital sensor 2 is put in a breast pocket of the clothes of the human body, the three detection axes X, Y and Z have an arrangement relation with respect to the human body as illustrated in FIG. 5B.

According to the arrangement relation, it is possible to detect a motion in a direction apart from (a direction close to) a human body breast as, for example, an acceleration on the detection axis Z. Further, in an attachment mode illustrated in FIG. 5B, the detection axis Z may be regarded to be orthogonal to the detection axis Y and be associated with a walking direction of the observed person.

The detection axis Z is oriented to a direction of directivity of a transmitted radio wave of the wireless sensor 21. The direction of the directivity of the transmitted radio wave of the wireless sensor 21 may be regarded to correspond to a direction of a detection axis of the wireless sensor 21.

Hence, one of the detection axes of the inertial sensor 22 may be oriented to detect an acceleration in the direction of the detection axis of the wireless sensor 21.

Detected values (gravitational acceleration [G]) of the detection axes X, Y and Z change according to a posture of the human body, so that the processor 31 can detect the posture of the human body based on the detected values of the detection axes X, Y and Z.

(Operation example)

Operation examples of the sensor system 1 according to one embodiment will be described below. In addition, an example where the information processing apparatus 3 processes sensor information will be described below as a non-restrictive example. In this regard, the sensor unit 2 (e.g. processor 23) may process the sensor information.

FIG. 6 is a flowchart illustrating an operation example of the sensor system 1 according to one embodiment. As illustrated in FIG. 6, when receiving an inertial sensor value from the sensor unit 2 (process P11), the information processing apparatus 3 (e.g. processor 31) may calculate an "extended wavelength" of the inertial sensor value (process P12).

The "extended wavelength" illustratively refers to a length in case where a signal waveform (in other words, a temporal change) of an inertial sensor value is linearly extended in a time domain. Hence, the "extended wavelength" is a concept different from a general "wavelength".

The "extended wavelength" may be regarded to correspond to a length of a locus traced by an inertial sensor value in a time domain per given unit time. In addition, the unit time may be a "second" unit or may be "minute" unit.

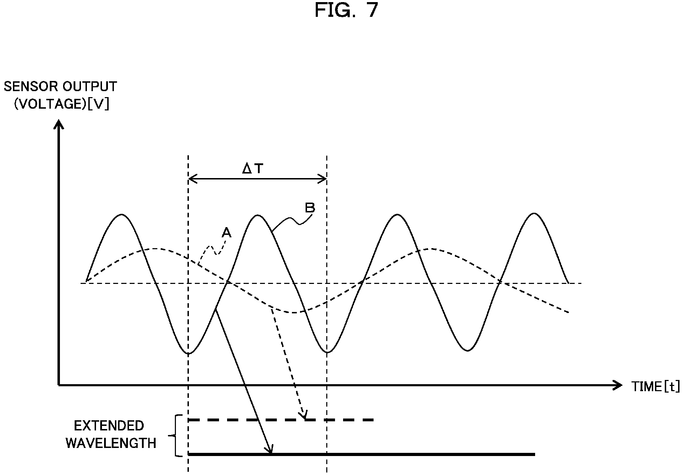

FIG. 7 schematically illustrates the concept of the "extended wavelength". A horizontal axis in FIG. 7 indicates a time (t), and a vertical axis in FIG. 7 indicates an inertial sensor value (e.g. a voltage [V]).

In FIG. 7, illustratively, a signal waveform indicated by a dotted line A schematically indicates a temporal change in an inertial sensor value in case where a sensing target observed person is in a resting state. Illustratively, a signal waveform indicated by a solid line B schematically indicates a temporal change in an inertial sensor value in case where the observed person is doing an activity.

As illustrated in a lower part in FIG. 7, the "extended wavelength" corresponds to a length in case where the signal waveforms per unit time ($\Delta$T) indicated by the dotted line A and the solid line B are linearly extended in the time direction.

The "extended wavelength" can be illustratively calculated by successively storing inertial sensor values in the storage unit 30 (see FIG. 4) at a given cycle (which may be referred to as a "sampling cycle") and adding a change amount of an amplitude value over a unit time.

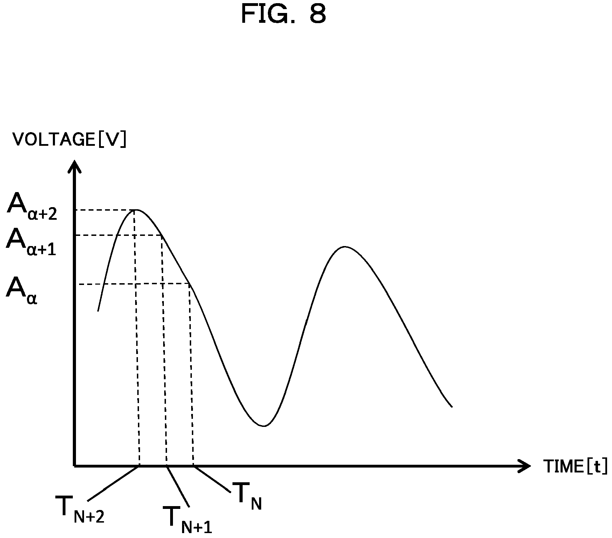

A calculation example of the "extended wavelength" will be described with reference to FIG. 8. A horizontal axis in FIG. 8 indicates a time (t), and a vertical axis in FIG. 8 indicates an inertial sensor value (e.g. a voltage [V] corresponding to an amplitude value).

In case of a signal waveform illustrated in FIG. 8, at given timings t = $T_{N+2}$, t = $T_{N+1}$ and t = $T_N$, inertial sensor values are "$A_{\alpha+2}$", "$A_{\alpha+1}$" and "$A_\alpha$".

In addition, "N" is an integer which represents a label of a timing. "A" is an actual number taken by a voltage value [V], and "$\alpha$" is an integer which represents a label of the voltage value. Each of the timings t = $T_{N+2}$, t = $T_{N+1}$ and t = $T_N$ may be referred to as a "sampling timing". A sampling timing interval may be fixed or be different.

The information processing apparatus 3 (e.g. processor 31) illustratively calculates an amplitude change amount between sampling timings based on an amplitude value (voltage value) obtained at each sampling timing. For example, the information processing apparatus 3 may calculate a difference between amplitude values of adjacent sampling timings as an amplitude change amount between the sampling timings.

Illustratively, the information processing apparatus 3 may calculate an amplitude change amount between a sampling timing t = $T_{N+2}$ and a next sampling timing t = $T_{N+1}$ as an absolute value $|A_{\alpha+1} - A_{\alpha+2}|$. Similarly, the information processing apparatus 3 may calculate an amplitude change amount between a sampling timing t = $T_{N+1}$ and a next sampling timing t - $T_N$ as an absolute value $|A_\alpha - A_{\alpha+1}|$.

The information processing apparatus 3 can calculate the "extended wavelength" by repeating such an arithmetic operation over a number of times of sampling per unit time, and adding the resulting amplitude change amount like $|A_\alpha - A_{\alpha+1}| + |A_{\alpha+1} - A_{\alpha+2}| + ....$

In addition, as illustrated in FIGS. 7 and 8, when the inertial sensor value is expressed by the voltage value [V], a unit of the "extended wavelength" may be expressed as "voltage/time" (V/min), for example.

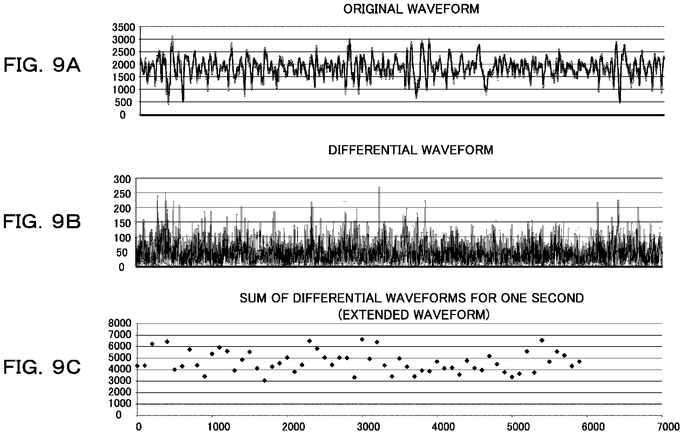

FIGS. 9A to 9C illustrate examples of an extended wavelength calculation process. FIG. 9A illustrates an original waveform of an inertial sensor value, FIG. 9B illustrates a differential waveform and FIG. 9C illustrates a value obtained by summing differential waveforms over a unit time (one second in this example).

The differential waveform illustrated in FIG. 9B represents an amplitude change amount per predetermined minute time, and is illustratively a differential waveform calculated at a sampling cycle of 1 kHz. Hence, the amplitude change amount illustratively represents an amplitude change amount per 1/1000.

The differential waveforms may be summed per second in the period $\Delta$ T illustrated in FIG. 7. For example, the amplitude change amounts may be summed every 1000 amplitude change amounts in the period $\Delta$T. Thus, as illustrated in FIG. 9C, the extended wavelength in the period $\Delta$T is calculated.

In addition, when the number of times of sampling of an amplitude value per unit time is too smaller, a calculation accuracy of "extended wavelength" lowers. When the number of times of sampling of an amplitude value per unit time is too larger, an arithmetic operation load is higher and arithmetic operation delay occurs. Therefore, a reasonable range may be set. Further, the "extended wavelength" may be time-averaged over a predetermined period of time. For example, 60 "extended wavelengths" obtained in one minute while the unit time is one second may be averaged.

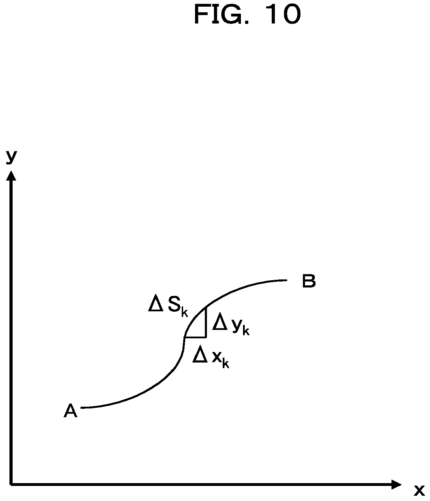

Further, the information processing apparatus 3 may calculate the "extended wavelength" as follows. For example, FIG. 10 illustrates an example where the "extended wavelength" of a curve AB is calculated. An interval between A and B is divided into n minute intervals, each minute interval is approximated as a line segment and a sum Sn of each length is expressed as in following equation (1).

[Equation 1]

$$Sn = \sum_{k=1}^{n} \Delta S_k \quad \ldots (1)$$

A minute displacement in each minute interval in an x direction is represented by $\Delta xk$ and a minute displacement in a y direction is represented by $\Delta yk$, $\Delta Sk$ is expressed by following equation (2) according to the Pythagorean theorem.

[Equation 2]

$$\Delta S_k = \sqrt{(\Delta x_k)^2 + (\Delta y_k)^2} \quad \ldots (2)$$

As indicated in following equation (3), when the number of minute intervals n in equation 2 is infinitely increased, the sum Sn approximates to the length L of the curve AB.

[Equation 3]

$$L = \lim_{n \to \infty} S_n = \lim_{n \to \infty} \sum_{k=1}^{n} \Delta x_k = \lim_{n \to \infty} \sum_{k=1}^{n} \sqrt{(\Delta x_k)^2 + (\Delta y_k)^2}$$

$$\ldots (3)$$

When the x direction is a time axis and a sampling cycle of an inertial sensor value is fixed (e.g. 1 kHz), "$x_k$" is fixed in equation (3), and, by substituting "$y_k$" with an inertial sensor value (amplitude value), the "extended wavelength" is calculated.

In addition, the "extended wavelength" of the inertial sensor value may be calculated for each of a plurality of detection axes (e.g. the X axis, the Y axis and the Z axis) of the inertial sensor 22.

The extended wavelength of the inertial sensor value is longer when the amplitude of the inertial sensor value is larger, and is longer when the frequency of the inertial sensor value is higher. Hence, the extended wavelength of the inertial sensor value tends to be longer when a motion of an observed person is greater and be longer when the motion of the observed person is faster. Consequently, the extended wavelength of the inertial sensor value can be used for an index of an exercise amount associated with a motion of an observed person.

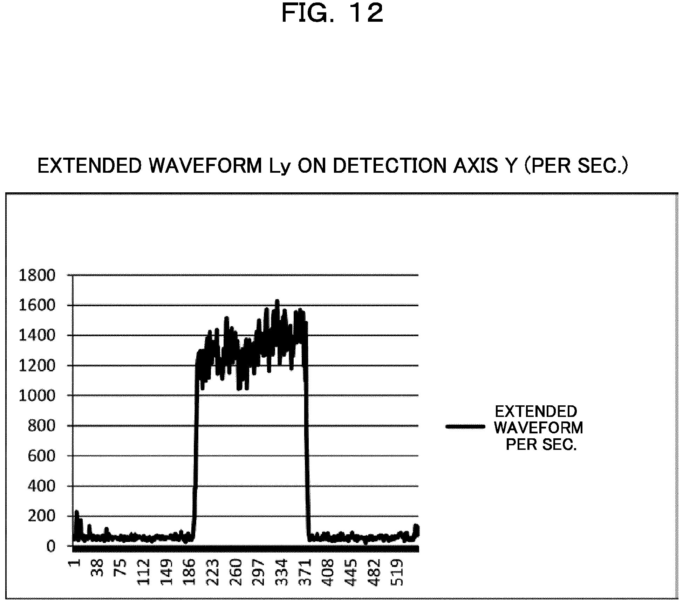

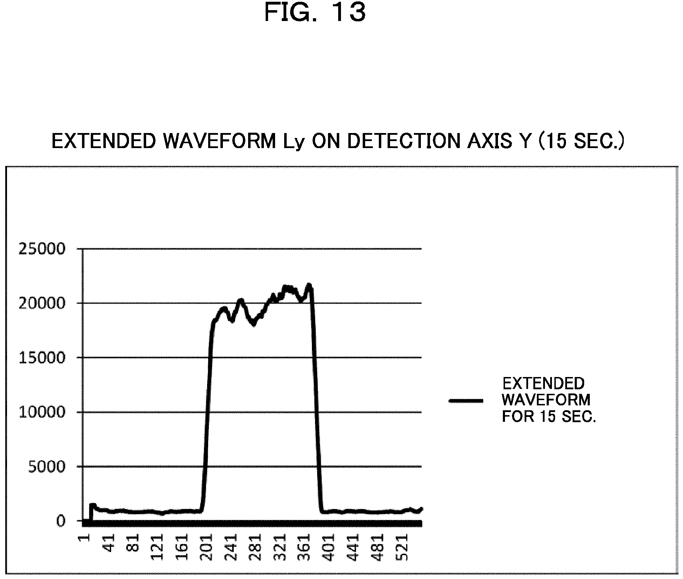

FIGS. 12 and 13 illustrate examples of an extended wavelength of an inertial sensor value. The extended wavelengths illustrated in FIGS. 12 and 13 correspond to an extended wavelength (Ly) of an inertial sensor value obtained on the detection axis Y of the inertial sensor 22 (see FIG. 5B).

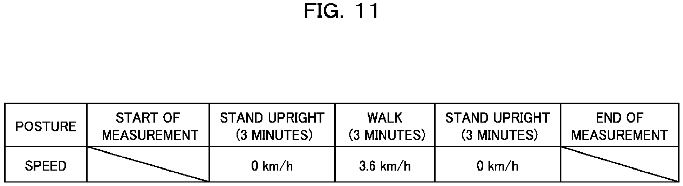

Further, the extended wavelength Ly illustrated in FIGS. 12 and 13 corresponds to an extended wavelength obtained according to a posture (which may be referred to as a "measurement condition" for descriptive purposes) taken by an observed person according to an activity from a start of measurement to an end of the measurement as illustrated in FIG. 11.

FIG. 11 illustrates a case where, from a start of measurement to an end of the measurement, the observed person "walks upright" 3.6 km per hour for three minutes from a state where the observed person "stands upright" for three minutes (0 km per hour), then stops walking and "stands upright" for three minutes (0 km per hour).

Under the measurement condition illustrated in FIG. 11, the extended wavelength Ly illustrated in FIG. 12 illustratively corresponds to an extended wavelength per second, and the extended wavelength Ly illustrated in FIG. 13 illustratively corresponds to an extended wavelength obtained by summing the 1-second extended wavelengths for 15 seconds.

As illustrated in FIGS. 12 and 13, the extended wavelength Ly of the inertial sensor value is longer during the three minutes during which the observed person "walks upright" than during the three minutes during which the observed person "stands upright" without walking.

When the extended wavelength Ly of the inertial sensor value is calculated, the information processing apparatus 3 may determine an activity state of the observed person based on the extended wavelength Ly as illustrated in FIG. 6 (process P13). In addition, an activity state determination cycle may be illustratively a 1-second cycle at which the above extended wavelength is obtained or a 15-second cycle.

(First example of activity state determination process)

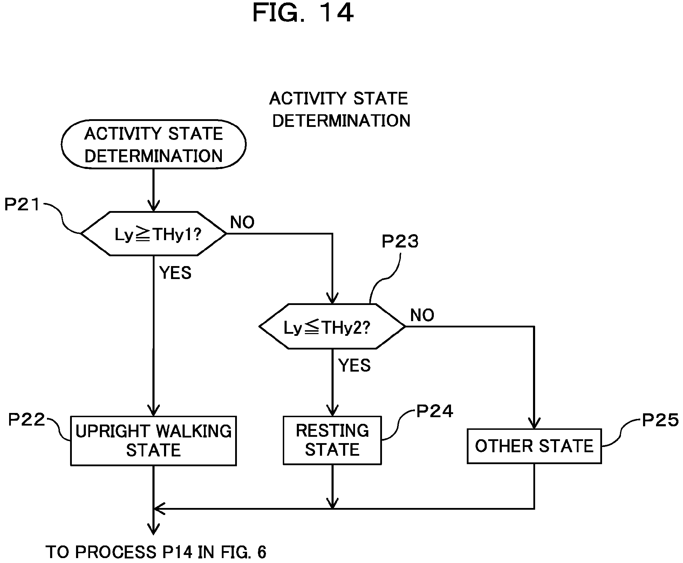

FIG. 14 illustrates the first example of the activity state determination process (P13). As illustrated in FIG. 14, the information processing apparatus 3 (e.g. processor 31) may compare the extended wavelength Ly and a threshold value THy1, and determine whether or not Ly ≥ THy1 holds (process P21).

The threshold value THy1 is an example of a first threshold value, and may be set to THy1 = 1000 as a non-restrictive example in case of the 1-second extended wavelength Ly and may be set to THy1 = 15000 in case of the 15-second extended wavelength Ly.

When a determination result indicates that Ly ≥ THy1 holds (YES in process P21), the information processing apparatus 3 may determine that the observed person is in an "upright walking" state (process P22).

Meanwhile, when Ly < THy1 holds (NO in process P21), the information processing apparatus 3 may compare the extended wavelength Ly and a second threshold value THy2 and determine whether or not Ly ≥ THy2 holds (process P23).

The threshold value THy2 is an example of a second threshold value and may be, for example, a value satisfying THy2 < THy1. The threshold value THy2 may be set to THy2 = 200 as a non-restrictive example in case of the 1-second extended wavelength Ly and may be set to THy2 = 5000 in case of the 15-second extended wavelength Ly.

When a determination result indicates that Ly ≤ THy2 holds (YES in process P23), the information processing apparatus 3 may determine that the observed person is in a "resting" state (process P24).

Meanwhile, when Ly > THy2 holds (NO in process P23), the information processing apparatus 3 may determine that the observed person is in a state different from each of the "upright walking" and "resting" states (process P25).

In addition, "another state" of a non-restrictive example includes a "forward-bent walking" state or a "stooping" activity state described below.

As described above, the information processing apparatus 3 can determine (classify in other words) an activity state of the observed person based on an extended wavelength of an inertial sensor value.

(Second example of activity state determination process)

The above determination and classification are based on the extended wavelength Ly on one detection axis Y of the inertial sensor 22. However, an activity state of the observed person may be determined and classified by using extended wavelengths of two or more detection axes of the inertial sensor 22 in a complex manner.

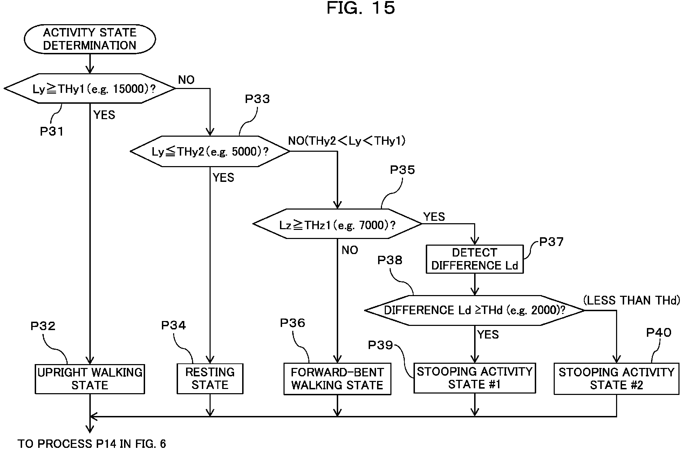

An example where an activity state of the observed person is determined based on the extended wavelengths Ly and Lz on the two detection axes Y and Z of the inertial sensor 22 will be described as a non-restrictive example with reference to FIGS. 15 to 18.

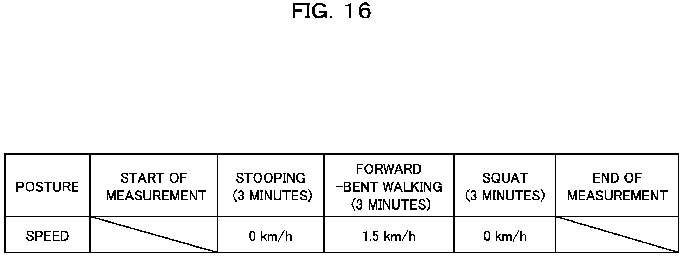

FIG. 15 is a flowchart illustrating a second example of the activity state determination process (P13) illustrated in FIG. 6, and FIG. 16 is a diagram illustrating another example of a measurement condition of the observed person from a start of measurement to an end of the measurement.

FIG. 16 illustrates an example as an non-restrictive example of the measurement condition where the observed person walks (which may be referred to as "forward-bent walking") 1.5 km per hour for three minutes at a forward-bent posture from a "stooping" state (0 km per hour) taken for three minutes and subsequently takes a "squat" posture for three minutes.

In addition, the "forward-bent walking" may illustratively correspond to a walk of the observed person in a forward-leaning state. For example, this walk corresponds to a case where the observed person walks in a forward-leaning state compared to an upright posture in case where the observed person climbs a steep slope.

Further, this walk may illustratively correspond to a walk in a state where an upper body is inclined in a stooping state such that the observed person takes a posture to bend a back or lower a head. For example, this walk corresponds to a case where the observed person walks lowering the head compared to a case where the observed person stands upright while hiding in shadows.

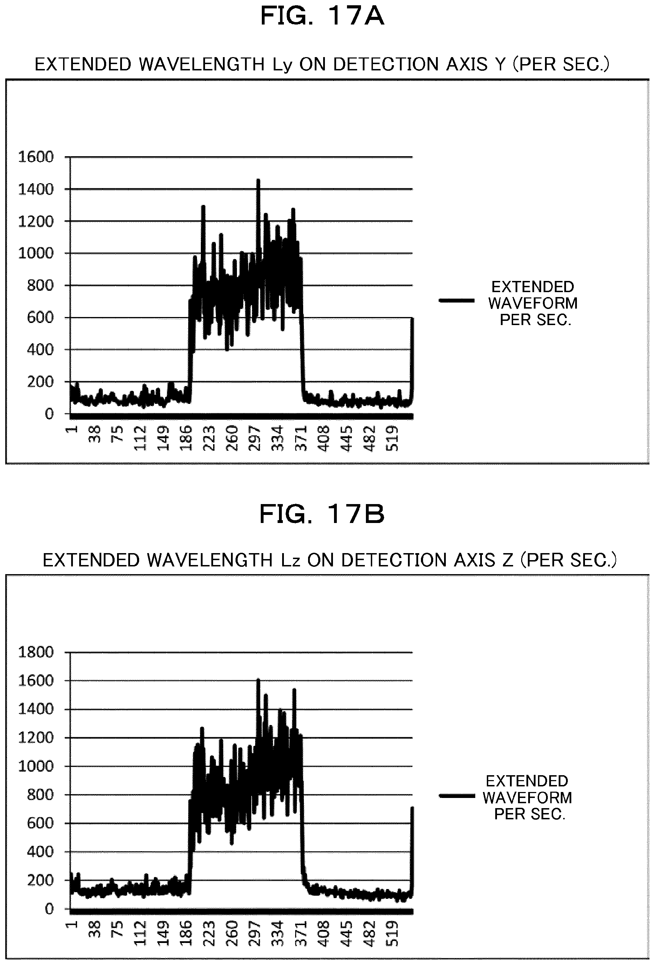

FIGS. 17A and 17B are diagrams illustrating examples of 1-second extended wavelengths obtained on the detection axes Y and Z under a posture condition illustrated in FIG. 16.

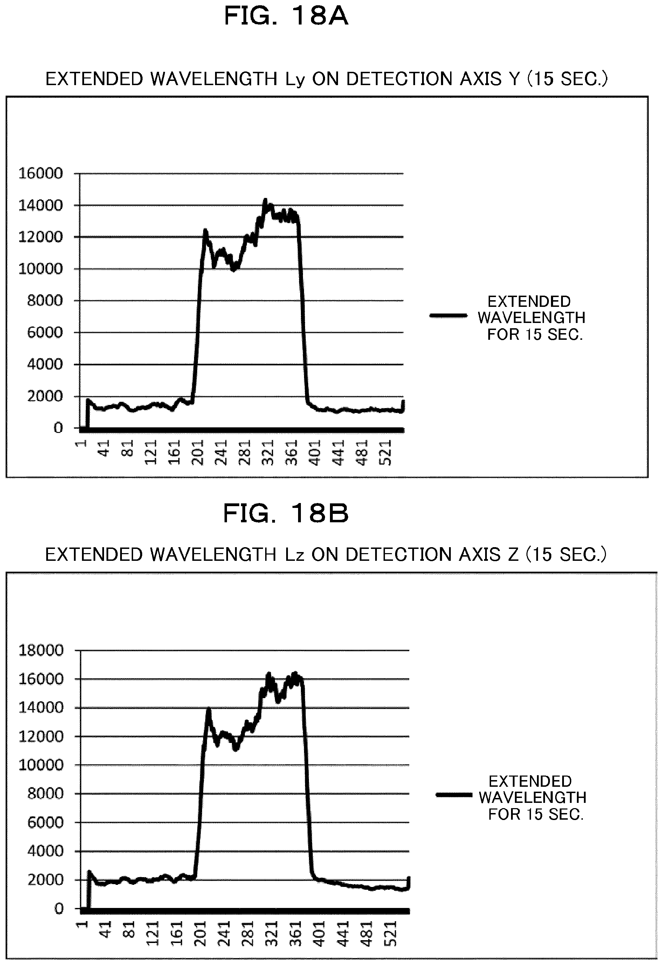

Further, FIGS. 18A and 18B are diagrams illustrating examples of 15-second extended wavelengths obtained on the detection axes Y and Z under the posture condition illustrated in FIG. 16.

As illustrated in FIG. 15, the information processing apparatus 3 (e.g. processor 31) may compare the extended wavelength $Ly$ and the threshold value $THy1$ on the detection axis Y, and determine whether or not $Ly \geq THy1$ holds (process P31).

The threshold value $THy1$ maybe set to $THy1 = 1000$ in case of the 1-second extended wavelength $Ly$ and may be set to $THy1 = 15000$ in case of the 15-second extended wavelength $Ly$.

When a determination result indicates that $Ly \geq THy1$ holds (YES in process P31), the information processing apparatus 3 may determine that the observed person is in an "upright walking" state (process P32).

Meanwhile, when $Ly < THy1$ holds (NO in process P31), the information processing apparatus 3 may compare the extended wavelength $Ly$ and the second threshold value $THy2$ similar to the first example, and determine whether or not $Ly \leq THy2$ holds (process P33).

The threshold value $THy2$ may be a value satisfying $THy2 < THy1$ similar to the first example. The threshold value $THy2$ may be set to $THy2 = 200$ as a non-restrictive example in case of the 1-second extended wavelength $Ly$ and may be set to $THy2 = 5000$ in case of the 15-second extended wavelength $Ly$.

When a determination result indicates that $Ly \leq THy2$ holds (YES in process P33), the information processing apparatus 3 may determine that the observed person is in a "resting" state (process P34).

Meanwhile, when $Ly > THy2$ holds (NO in process P33), the information processing apparatus 3 may compare the extended wavelength $Lz$ and a threshold value $THz1$ on the detection axis Z, and determine whether or not $Lz \geq THz1$ holds (process P35).

The threshold value $THz1$ is an example of a third threshold value, and may be illustratively a value satisfying $THy2 < THz1 < THy1$. The threshold value $THz1$ maybe set to $THz1 = 400$ as a non-restrictive example in case of the 1-second extended wavelength $Ly$ and may be set to $THz1 = 7000$ in case of the 15-second extended wavelength $Ly$.

When a determination result indicates that $Lz < THz1$ holds (NO in process P35), the information processing apparatus 3 may illustratively determine that the observed person is in a "forward-bent walking" state (process P36).

Meanwhile, when $Lz \geq THz1$ holds (YES in process P35), the information processing apparatus 3 may assume the extended wavelengths $Lx$, $Ly$ and $Lz$ on the detection axes X, Y and Z, and calculate the difference $Ld$ by subtracting a following difference $Ld1$ and difference $Ld2$ (process P37) .

$$Ld1 = Lz - Lx$$

$$Ld2 = Ly - Lx$$

$$Ld = Ld1 - Ld2 = Lz - Ly$$

When the difference $Ld$ which is a subtraction result of $Ld1$ and $Ld2$ is calculated, the information processing apparatus 3 may illustratively compare the difference $Ld$ and a threshold value $THd$ and determine whether or not $Ld \geq THd$ holds (process P38).

The threshold value $THd$ is an example of a fourth threshold value, and may be illustratively a value satisfying $THd < THy2$. The threshold value $THd$ may be set to $THd = 150$ as a non-restrictive example in case of the 1-second extended wavelength and may be set to $THd = 2000$ in case of the 15-second extended wavelength.

When a determination result indicates that $Ld \geq THd$ holds (YES in process P38), the information processing apparatus 3 may illustratively determine that the observed person is in a first state #1 (also abbreviated as a "stooping activity state #1" for descriptive purposes) where the observed person does an activity while stooping (process P39).

Meanwhile, when $Ld < THd$ holds (NO in process P38), the information processing apparatus 3 may illustratively determine that the observed person is in a second state #2 (also abbreviated as a "stooping activity state #2" for descriptive purposes) where the observed person does an activity while stooping (process P40).

A difference between the stooping activity states #1 and #2 is caused by whether a difference $Ld = Lz - Ly$ is large or small. According to, for example, the relation between the detection axes X, Y and Z illustrated in FIG. 5B, when the difference $Ld$ is greater, the motion of the vital sensor 2 is relatively greater in the Z axis direction than in the Y axis direction.

By contrast with this, when the difference $Ld$ is smaller, the motion of the vital sensor 2 is relatively greater in the Y axis direction than in the Z axis direction.

Hence, the degree of "stooping" is greater in the stooping activity state #1 than in the stooping activity state #2.

As a non-restrictive example, the stooping activity state #1 can be associated with a state where the observed person picks up trash or does weeding, and the stooping activity state #2 can be associated with a state where the observed person mops or vacuums.

As described above, according to the first example and the second example of the activity state determination processing, it is possible to determine and classify an activity state of the observed person based on an extended wavelength of an inertial sensor value.

In this regard, the extended wavelength of the inertial sensor value differs from an instantaneous inertial sensor value at a given timing or a time average value of inertial sensor values over a given period of time, and is information indicating the amount of movement of the inertial sensor 22 per unit time (in other words, the amount of movement of the observed person).

Hence, the extended wavelength of the inertial sensor value has an improved noise resistance compared to a case where an instantaneous inertial sensor value or a time average value of the inertial sensor value is compared with a reference value (threshold value). Since the noise resistance improves, a determination accuracy of an activity state of the observed person also improves.

Further, even when the vital sensor 2 is put in a pocket of the clothes of the observer person and an asynchronous motion is made between the vital sensor 2 and the observed person, it is possible to detect quantitatively the amount of movement of the observed person based on the extended wavelength of the inertial sensor value. Consequently, it is possible to improve a determination accuracy of an activity state of the observed person.

Further, the extended wavelength can be calculated with a smaller arithmetic operation amount compared to a case where a sensor value of a given time interval is integrated to calculate an area or a determinant such as an affine matrix is calculated. Consequently, it is possible to reduce a processing load of the information processing apparatus 3 and improve a processing speed, for example.

In addition, the above threshold values THy1, THy2, THz1 and THd may be illustratively stored in the storage unit 30 of the information processing apparatus 3. When the processor 23 of the sensor unit 2 executes the processes illustrated in FIGS. 14 and 15, the threshold values THy1, THy2, THz1 and THd may be stored in the memory 24 of the sensor unit 2, for example.

(Selection and determination of heartbeat detection algorithm)

When an activity state of the observed person is determined and classified as in the first example or the second example of the above-described activity state determination process, the information processing apparatus 3 may adaptively select and determine the "heartbeat detection algorithm" matching the determined and classified activity state as illustrated in FIG. 6 (process P14).

The heartbeat detection algorithm (which may be paraphrased as a "method" or a "process") is illustratively an algorithm of detecting a heart rate of the observed person based on a wireless sensor value or a wireless sensor value and an inertial sensor value.

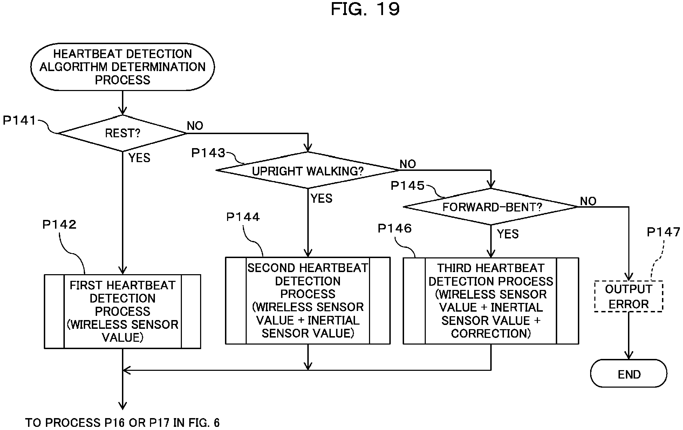

As a non-restrictive example, when an activity state determination result is "resting" state as illustrated in FIG. 19 (YES in process P141), the information processing apparatus 3 may select a first heartbeat detection algorithm (process P142).

The first heartbeat detection algorithm is an example of the heartbeat detection process based on the wireless sensor value, and a detailed process example will be described below.

Meanwhile, when the activity state determination result indicates "upright walking" (NO in process P141 and YES in process P143), the information processing apparatus 3 may select a second heartbeat detection algorithm (process P144).

The second heartbeat detection algorithm is an example of the heartbeat detection process based on the wireless sensor value and the inertial sensor value, and a detailed process example will be described below.

Further, when the activity state determination result indicates "forward-bent walking" or a "stooping activity state" (NO in process P143 and YES in process P145), the information processing apparatus 3 may select a third heartbeat detection algorithm (process P146).

The third heartbeat detection algorithm is an example of a heartbeat detection process based on the wireless sensor value and the inertial sensor value similar to the second heartbeat detection algorithm. In this regard, the third heartbeat detection algorithm differs from the second heartbeat detection algorithm in including a process of correcting a detection sensitivity of the wireless sensor 21. A detailed process example of the third heartbeat detection algorithm will be described below.

In addition, when the activity state determination result indicates none of "resting", "upright walking", "forward-bent walking" and the "stooping activity state" (NO in processes P141, P143 and P145), the information processing apparatus 3 may finish the process.

Before finishing the process, the information processing apparatus 3 may output error information to the output device such as the display or the printer as indicated by a dotted line in FIG. 19 (process P147).

The information processing apparatus 3 may detect the heart rate of the observed person according to the heartbeat detection algorithm selected and determined as described above (process P15 in FIG. 6).

The calculated heart rate may be used as a parameter to control a spatial environment in which the observed person is positioned (process P16 in FIG. 6). Further, the calculated heart rate may be optionally outputted to an output device such as the display or the printer as indicated by a dotted line in FIG. 6 (process P17 om FIG. 6).

In addition, information of the extended wavelength calculated in process P12, the activity state determination result in process P13, information of the heartbeat detection algorithm determined in process P14 and information indicating a status of the heartbeat detection process in process P15 may be optionally outputted to the output device such as the display or the printer.

In this case, for example, the calculated extended wavelength, the activity state determination status and the heartbeat detection process status can be checked by using the output device.

As described above, the information processing apparatus 3 adaptively selects and determines (which may be referred to as "change") the heartbeat detection algorithm according to an activity state determination result of the observed person and, consequently, detect a heartbeat of the observed person according to the heartbeat detection algorithm matching the activity state of the observed person.

For example, an appropriate heartbeat detection algorithm is applicable to detect the heartbeat of the observed person according to whether or not the observed person makes a motion or the degree of motion.

In this regard, the activity state determination result of the observed person based on which the heartbeat detection algorithm is selected and determined is based on the "extended wavelength" of a inertial sensor value as described above and is obtained with improved accuracy. Consequently, an accuracy to select and determine the heartbeat detection algorithm also improves.

(Example of heartbeat detection process)

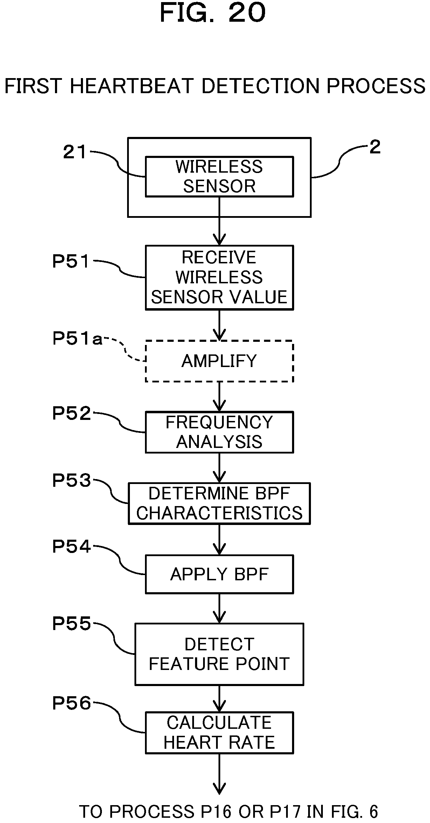

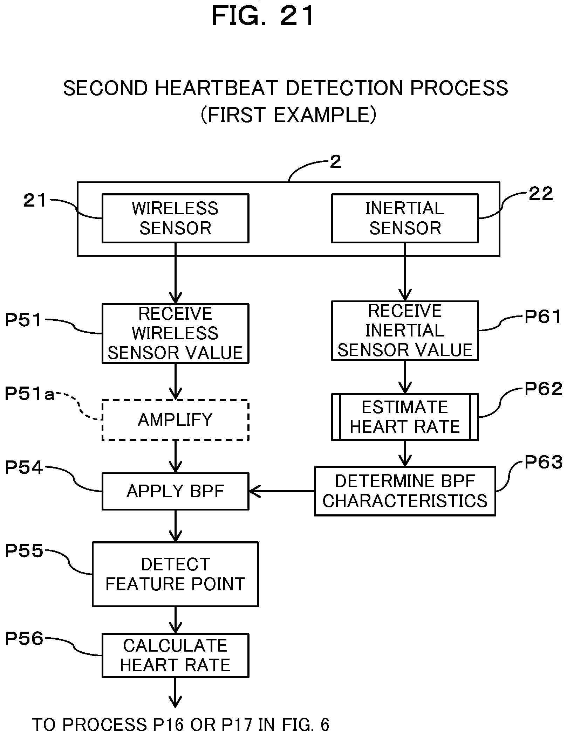

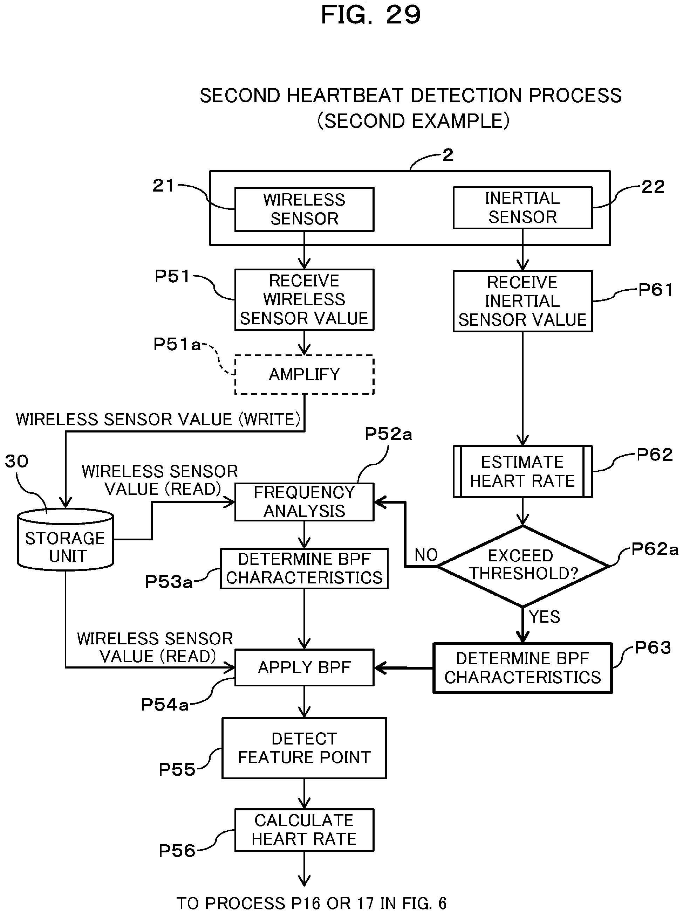

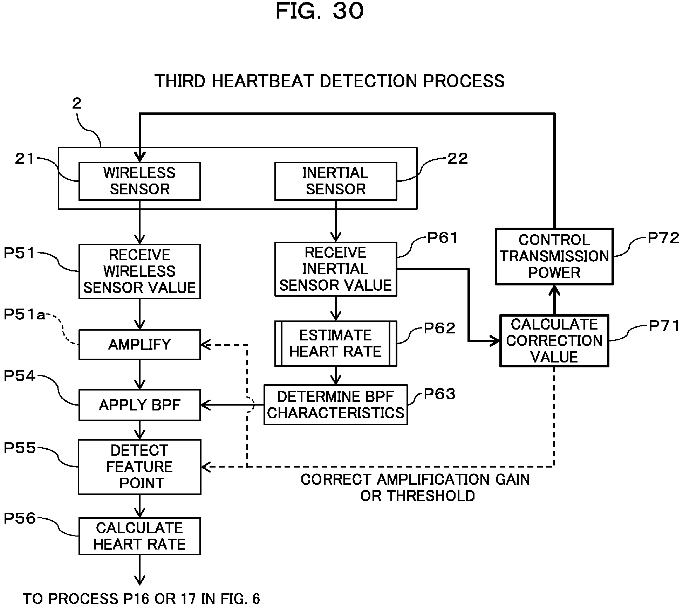

Next, an example of the first heartbeat detection process illustrated in FIG. 19 will be described with reference to FIG. 20, and an example of the second heartbeat detection process illustrated in FIG. 19 will be described with reference to FIGS. 21 and 29. Next, an example of the third heartbeat detection process illustrated in FIG. 19 will be described with reference to FIG. 30.

(Example of first heartbeat detection process)

FIG. 20 is a flowchart illustrating an example of the first heartbeat detection process according to one embodiment. The flowchart illustrated in FIG. 20 may be illustratively executed by the processor 31 of the information processing apparatus 3.

As illustrated in FIG. 20, when determining that the observed person is in the "resting" state and selecting and determining the first heartbeat detection process, the information processing apparatus 3 may analyze the frequency of the wireless sensor value received from the vital sensor 2 (processes P51 and P52). When determining that the observed person is in a "resting" state, the information processing apparatus 3 does not need to use an inertial sensor value for the heartbeat detection process.

The received wireless sensor value may be stored in the storage unit 30 (see FIG .4). Further, the received wireless sensor value may be optionally amplified as indicated by a dotted line in FIG. 20 (process P51a) or does not need to be amplified. The wireless sensor value does not need to be amplified likewise in processes described below with reference to FIGS. 21 and 29.

For frequency analysis of a wireless sensor value, fast Fourier transform (FFT) or discrete Fourier transform (DFT) may be illustratively used. The wireless sensor value is converted from a time domain signal into a frequency domain signal (which may be referred to as a "frequency signal" for descriptive purposes) by FFT or DFT.

The information processing apparatus 3 may detect, for example, a frequency having a peak value (such a frequency may be referred to as a "peak frequency") in a frequency signal of the wireless sensor value. The peak frequency is an example of a frequency component indicating a characteristic change corresponding to a heartbeat.

The information processing apparatus 3 may determine characteristics of a bandpass filter (BPF) (which may be abbreviated as "BPF characteristics") applied to a wireless sensor value, based on the detected peak frequency (process P53).

As a non-restrictive example, the information processing apparatus 3 may determine BPF characteristics which have the peak frequency at the center frequency and a narrower bandwidth when the peak frequency is higher (a wider bandwidth when the peak frequency is lower) as the BPF characteristics applied to the wireless sensor value. A reason that a narrower BPF bandwidth is set and controlled when the peak frequency is higher, and a detailed determination example of variable BPF characteristics will be described below.

The information processing apparatus 3 may apply the BPF of the BPF characteristics determined in process P53, to the wireless sensor value (process P54). The wireless sensor value to which the BPF is applied may be optionally read from the storage unit 30.

Applying the BPF can cancel or suppress a noise component which is a non-heartbeat derived signal component of the observed person (e.g. body motion derived signal component of the observed person) from the wireless sensor value.

The information processing apparatus 3 may detect a heartbeat component indicative a distinctive change corresponding to a heartbeat of the observed person as a "feature point" from the BPF-applied wireless sensor value (process P55). The "feature point" may be, for example, a point at which a first derivation becomes zero in a signal waveform of the BPF-applied wireless sensor value.

In response to detection of the "feature point", the information processing apparatus 3 can calculate a heart rate of the observed person per minute by calculating a time interval (e.g. "second") at the feature point and dividing one minute (= 60 seconds) by the calculated time interval (process P56).

As described above, according to the first heartbeat detection process example, when the observed person is in the "resting" state, it is possible to effectively suppress a noise component which is a non-heartbeat derived signal component of the observed person in a wireless sensor value by applying the BPF of the variable BPF characteristics to the wireless sensor value. Consequently, it is possible to improve a detection accuracy of heart rate when the observed person is in the "resting" state.

(First example of second heartbeat detection process example)

FIG. 21 is a flowchart illustrating the first example of the second heartbeat detection process according to one embodiment. The flowchart illustrated in FIG. 21 may be illustratively executed by the processor 31 of the information processing apparatus 3.

As illustrated in FIG. 21, when determining that the observed person is in the "upright walking" state and selecting and determining the second heartbeat detection process, the information processing apparatus 3 (e.g. processor 31) may estimate the heart rate of the observed person based on the inertial sensor value received from the vital sensor 2 (processes P61 and P62). In addition, the received inertial sensor value may be stored in the storage unit 30 (see FIG .4).

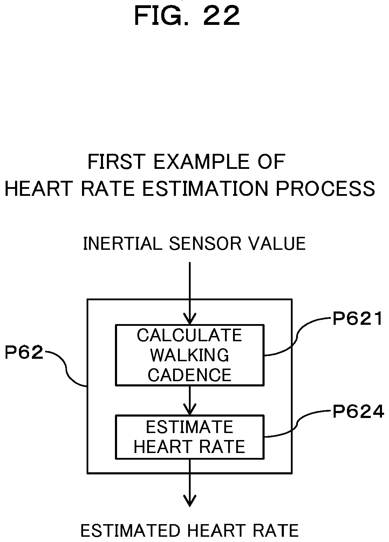

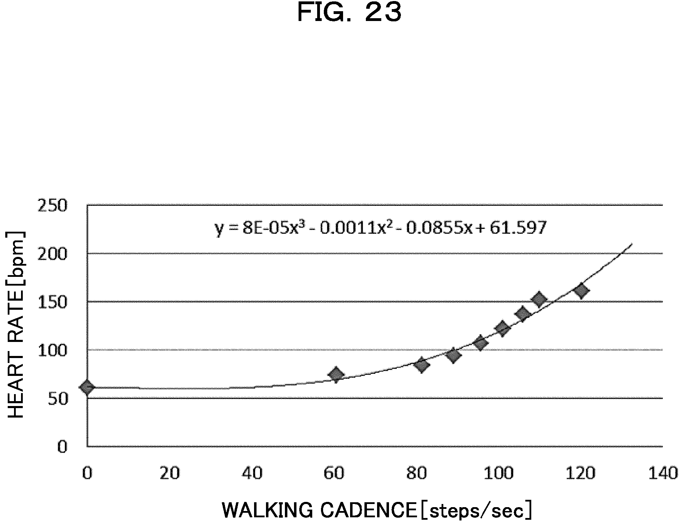

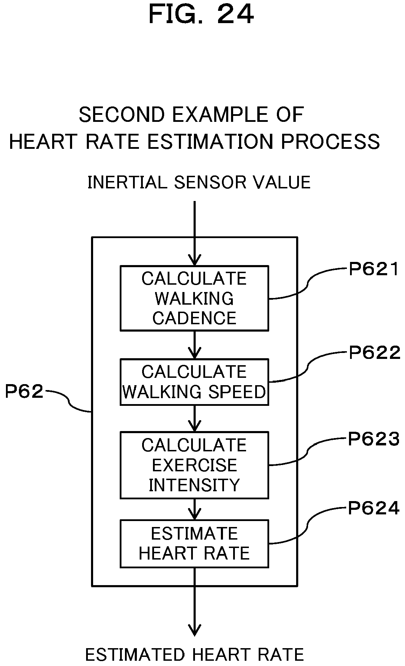

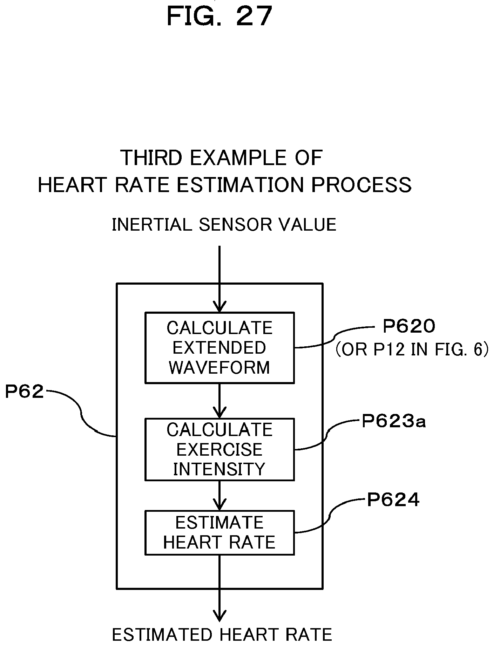

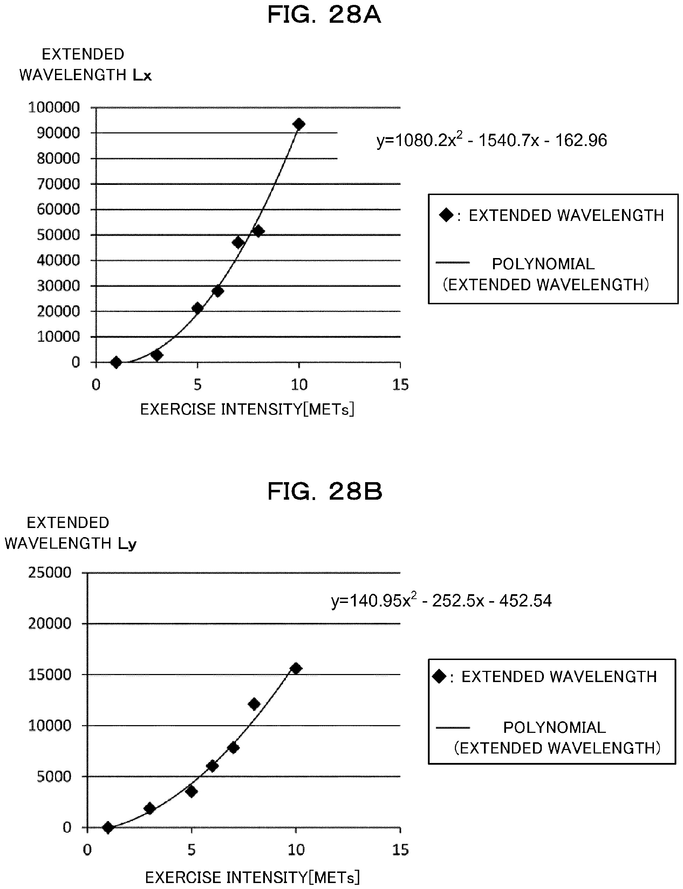

FIGS. 22 and 23 illustrate a first example of a heart rate estimation process, FIGS. 24 to 26 illustrate a second example of the heart rate estimation process, and FIGS. 27 and 28 illustrate a third example of the heart rate estimation process.

(First example of heart rate estimation process)

As illustrated in FIG. 22, the information processing apparatus 3 may calculate the number of steps (which may be referred to as a "walking cadence") of the observed person per unit time (illustratively one second) based on an inertial sensor value (process P621). Similar to general pedometers, the number of steps can be obtained by, for example, counting in the processor 31 the number of times that the inertial sensor value exceeds a threshold value.

Here, the walking cadence and the heart rate can be linked by a relational equation. The relational equation may be illustratively derived by performing a curve-fitting on a plurality of actual measured values, for example.

A non-restrictive example of the relational equation of the walking cadence and the heart rate can be expressed by following polynominal equation (4) as illustrated in FIG. 23.

$$y = 8E\text{-}05x^3 - 0.0011x^2 - 0.0855x + 61.597 \ldots (4)$$

In FIG. 23, a horizontal axis "x" indicates a walking cadence [the number of steps/second], and a vertical axis "y" indicates a heart rate per unit time (for example, one minute) [beats per minute, bpm]. Further, "8E-05" represents "$8 \times 10^{-5}$".

The information processing apparatus 3 may calculate the heart rate by calculating equation (4) based on the walking cadence calculated in process P621 (process P624 in FIG. 22). The calculated heart rate may be referred to as an "estimated heart rate" for descriptive purposes.

The relation illustrated in FIG. 23 may be expressed by data in a table format (which may be referred to as "table data"), for example. The table data may be stored in the storage unit 30 (see FIG. 4) of the information processing apparatus 3, for example. The information processing apparatus 3 may find the estimated heart rate with respect to the walking cadence by referring to the table data without performing an arithmetic operation.

(Second example of heart rate estimation process)

Next, the second example of the heart rate estimation process (P22) in FIG. 21 will be described with reference to FIGS. 24 to 26. The above first example of the heart rate estimation process is an example where a heart rate is calculated based on a walking cadence of the observed person. In the second example, the heart rate may be calculated based on an exercise intensity of the observed person.

As illustrated in FIG. 24, similar to the first example (FIG. 22), the information processing apparatus 3 (e.g. processor 31) may calculate the walking cadence of the observed person based on the wireless sensor value (process P621).

The information processing apparatus 3 may calculate a walking speed based on the walking cadence according to the calculated walking cadence (process P622).

In this regard, the walking cadence and the walking speed can be linked by a relational equation. The relational equation may be derived by performing a curve-fitting on a plurality of actual measured values.

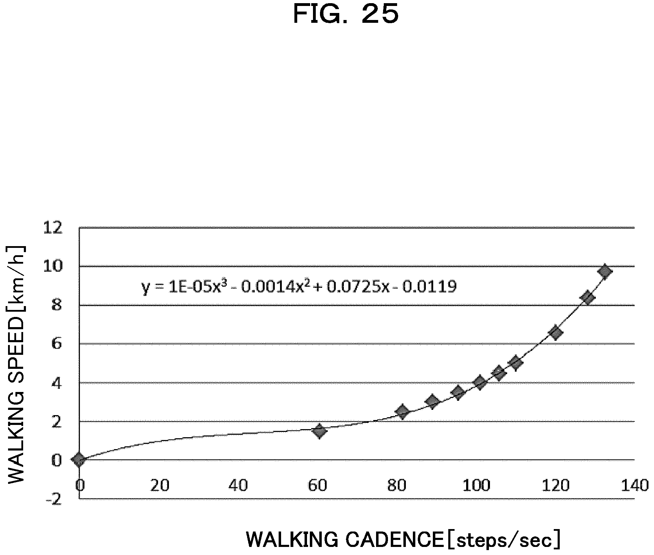

A non-restrictive example of the relational equation of the walking cadence and the walking speed can be expressed by following polynominal equation (5) as illustrated in FIG. 25.

$$y=1E-05x^3-0.0014x^2+0.0725x-0.0119...(5)$$

In FIG. 25, a horizontal axis "x" indicates a walking cadence [the number of steps/second], and a vertical axis "y" indicates a walking speed [km/h] per unit time (illustratively, one hour). Further, "1E-05" represents "$1x10^{-5}$".

As a walking distance per unit time increases, a step width of a distance per step and the number of steps also tend to increase. For example, a step width = height x 0.37 is applicable when the user walks 70 m per minute (= 4.2 km per hour), a step width = height x 0.45 is applicable when the user walks 90 m per minute (= 5.4 km per hour) and a step width = height x 0.5 is applicable when the user walks 110 m per minute (= 6.6 km per hour). Therefore, it is possible to approximately calculate the walking speed corresponding to a walking distance per unit time based on the above step width and walking cadence.

The information processing apparatus 3 may calculate the walking speed by calculating equation (5) based on the walking cadence calculated in process P621.

In addition, the relation illustrated in FIG. 25 may be expressed by, for example, table data similar to the relation illustrated in FIG. 23, and be stored in the storage unit 30 (see FIG. 4). The information processing apparatus 3 may find the walking speed with respect to the walking cadence by referring to the table data without performing an arithmetic operation.

The information processing apparatus 3 may calculate an exercise intensity of the user based on the walking speed according to the calculated walking speed (process P623 in FIG. 24). The exercise intensity is an index value indicative of an activity amount of a person, and may be expressed by a METs value.

METs is an abbreviation of "Metabolic equivalents". The METs value maybe a numerical value which expresses a relative value (e.g. a multiple number) of a metabolic rate (or a calorie consumption amount) during an activity of a person with respect to a metabolic rate during rest. A table in which METs values are associated with each activity of a person is referred to as a "METs table". The METs table is published by the National Institute of Health and Nutrition, for example.

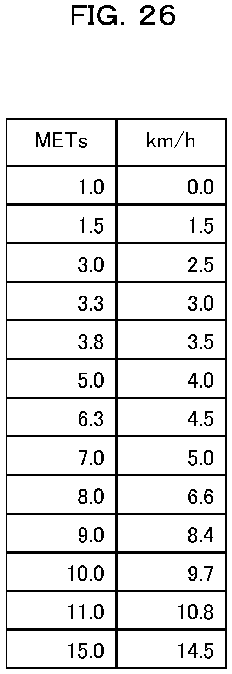

FIG. 26 illustrates an example of a relation between walking speeds and METs values. A walking speed = 0 [km/h] is associated with a METs value = 1 and corresponds to a reference exercise intensity during rest. As illustrated in FIG. 26, as the walking speed increases, the METs value also increases.

For example, in case of a walking speed = 2.5 [km/h], the METs value is three times as a reference METs value (=1). In case of a walking speed = 4 [km/h], the METs value is five times as the reference METs value.

When the METs value is determined, a current heart rate can be determined based on an age of the user and the heart rate during rest, for example. The METs value can be expressed by following equation (6), for example.

$$\text{METs value} = (\text{heart rate} - \text{heart rate during rest}) / (\text{maximum heart rate} - \text{heart rate during rest}) \times 10 ... (6)$$

A predetermined value may be used for a rest-time heart rate and a maximum heart rate. The predetermined value may be set as an average heart rate of a human body or may be set as an actual measured value per observed person. The "maximum heart rate" is simply calculated as "220 - age".

Consequently, the information processing apparatus 3 can calculate the current heart rate based on the METs value according to equation (6) (process P624 in FIG. 24). In other words, even when it is difficult to accurately detect a heart rate during an exercise or an activity of the user, it is possible to estimate a heart rate based on the METs value by calculating equation (6).

(Third example of heart rate estimation process)

Next, the third example of the heart rate estimation process (P22) in FIG. 21 will be described with reference to FIGS. 27 and 28. The exercise intensity calculated in the second example of the heart rate estimation process (see process P623 in FIG. 24) may be calculated based on an extended wavelength of an inertial sensor value.

As illustrated in, for example, FIG. 27, the information processing apparatus 3 (e.g. processor 31) may calculate an extended wavelength of an inertial sensor value (process P620), and calculate an exercise intensity of the observed person based on a relation between the calculated extended wavelength and the exercise intensity (process P623a).

In addition, extended wavelength calculation process P620 in FIG. 27 may correspond to extended wavelength calculation process P12 illustrated in FIG. 6. In other words, the extended wavelength calculated in process P12 may be used to calculate the exercise intensity in process P623.

The extended wavelength of the inertial sensor value may be calculated for each of a plurality of detection axes of the inertial sensor 22 as described above.

The extended wavelength of the inertial sensor value and the exercise intensity can be associated according to a given relational equation. The relational equation may be illustratively derived by performing a curve-fitting (in other words, fitting to a curve) on a plurality of actual measured values.

As a non-restrictive example, the relational equations of the extended wavelength of the inertial sensor value and the exercise intensity in directions of the detection axes X and Y illustrated in FIG. 5B can be expressed by polynomials (7) and (8) as illustrated in FIGS. 28A and 28B.

$$y=1080.2x^2-1540.7x-162.96\ldots(7)$$

$$y=140.95x^2+252.58x-452.54\ldots(8)$$

In addition, horizontal axes "x" in FIGS. 28A and 28B indicate the exercise intensity (METs), a vertical axis "y" in FIG. 28A indicates the extended wavelength on the detection axis X and a vertical axis "y" in FIG. 28B indicates the extended wavelength on the detection axis Y.

The information processing apparatus 3 may calculate the exercise intensities of the observed person on the detection axes X and Y by, for example, calculating equation (7) and equation (8) based on the extended wavelength calculated in process P620 (process P623a).

In addition, the relations illustrated in FIGS. 28A and 28B may be expressed by table data, for example. The table data may be stored in a storage unit 30 (see FIG. 4) of the information processing apparatus 3, for example. The information processing apparatus 3 may find the exercise intensity with respect to the extended wavelength by referring to the table data without performing an arithmetic operation.

When the exercise intensity is found, the information processing apparatus 3 may calculate a heart rate of the observed person based on the exercise intensity similar to the second example of the heart rate estimation process (see FIGS. 24 to 26) (process P624). For example, the exercise intensity may be calculated based on a sum of extended wavelengths on the detection axis X, the detection axis Y and the detection axis Z.

By using an "extended wavelength" of an inertial sensor value which can quantitatively express a motion amount of the observed to estimate a heart rate of the observed person, a noise resistance improves, and an estimation accuracy of heart rate also improves. Consequently, following accuracy to determine characteristics of the BPF applied to a wireless sensor value also improves.

When the estimated heart rate is calculated as in the first example, the second example or the third example of the above heart rate estimation process, the information processing apparatus 3 may determine characteristics of a BPF applied to a wireless sensor value and matching the estimated heart rate as (process P63 in FIG. 21).

For example, the information processing apparatus 3 may convert the estimated heart rate into a frequency, and set the converted frequency to a center frequency of the BPF applied to the wireless sensor value. The heart rate can be converted into the frequency by dividing the heart rate by 60 (seconds), for example. Further, the information processing apparatus 3 may set a narrow bandwidth to the BPF when the estimated heart rate is higher similar to the first heartbeat detection process (FIG. 20).

The information processing apparatus 3 may apply the BPF of the determined BPF characteristics to the wireless sensor value received in process P51, and filter the wireless sensor value (process P54 in FIG. 21). This filtering can cancel, for example, a body motion derived noise component which is a non-heartbeat derived signal component of the observed person, from the wireless sensor value.

The information processing apparatus 3 may detect a "feature point" from the BPF applied wireless sensor value similar to the first heartbeat detection process (process P55 in FIG. 21), and calculate a heart rate of the observed person based on the detected "feature point" (process P56 in FIG. 21).

As described above, according to the second heartbeat detection process example, the characteristics of the BPF applied to a wireless sensor value are controlled according to a frequency associated with a heart rate estimated based on an inertial sensor value when the observed person is in an "upright walking" state.

Consequently, it is possible to efficiently cancel a noise component appearing in a frequency band (which may be referred to as a "heartbeat appearance band" for descriptive purposes) in which a heartbeat component is assumed to appear on average in the wireless sensor value. Consequently, it is possible to improve a detection accuracy of heartbeat signal based on the wireless sensor value.

For example, it is possible to improve a detection accuracy of a heartbeat signal in a band of 1.5 to 4.0 Hz which is considered as being easily influenced by a body motion derived noise component within a band of 0.8 to 4.0 Hz which is an example of the heartbeat appearance band.


(Second example of second heartbeat detection process)

In the first example of the second heartbeat detection process, BPF characteristics are determined based on an estimated heart rate irrespectively of whether the heart rate estimated based on an inertial sensor value is high or low. However, the BPF characteristics may be determined based on a wireless sensor value without depending on the inertial sensor value in accordance with an estimated heart rate.

When, for example, a heart rate estimated based on an inertial sensor value or an "extended wavelength" of the inertial sensor value is high, a METs value which is an index of an associated exercise intensity also tends to be high. Hence, when the estimated heart rate, a METs value or the extended wavelength of the inertial sensor value exceeds the threshold value, the BPF characteristics may be determined based on the heart rate estimated based on the inertial sensor value as in the first example.

When the estimated heart rate is higher, in other words, when the METs value is higher or the extended wavelength of the inertial sensor value is longer, it is said that a body motion derived noise component is likely to be mixed in a heartbeat appearance band of the wireless sensor value.

By contrast with this, when the heart rate estimated based on the inertial sensor value, the METs value or the extended wavelength of the inertial sensor value is less than the threshold value, even when the BPF characteristics are determined based on the wireless sensor value, it is possible to sufficiently cancel the body motion derived noise component.

In other words, when the heart rate estimated based on the inertial sensor value, the METs value or the "extended wavelength" of the inertial sensor value is less than the threshold value, an influence on detection of a heartbeat component caused by the body motion derived noise component may be regarded to be little in some cases.

Hence, in the second example of the second heartbeat detection process, the information processing apparatus 3 (e.g. processor 31) may execute the flowchart illustrated in FIG. 29.

Illustratively, the information processing apparatus 3 may perform control based on which one of the inertial sensor value and the wireless sensor value BPF characteristics are determined by performing a threshold determination on the estimated heart rate (or the METs value or the "extended wavelength" of the inertial sensor which applies likewise below) (process P62a).

For example, according to determination as to whether or not the estimated heart rate exceeds the threshold value (YES in process P62a), the information processing apparatus 3 (e.g. processor 31) may determine the BPF characteristics based on a frequency associated with the heart rate estimated based on the inertial sensor value (process P63). The frequency corresponding to the heart rate may be referred to as a "heartbeat frequency" for descriptive purposes. The "threshold value" used in process P62a may be illustratively stored in the storage unit 30.

Meanwhile, according to the determination that the estimated heart rate is the threshold value or less (NO in process P62a), the information processing apparatus 3 may detect the heart rate based on the wireless sensor value, and determine the BPF characteristics based on the detected heartbeat frequency (processes P52a and P53a).

Frequency analysis (process P52a) may be used to detect a heart rate based on a wireless sensor value similar to the first heartbeat detection process (see, for example, FIG. 20). FFT or DFT may be used for the frequency analysis. The wireless sensor value is converted from a time domain signal into a frequency domain signal by FFT or DFT.

The information processing apparatus 3 may detect, for example, a peak frequency having a peak value in the frequency signal of the wireless sensor value, and the information processing apparatus 3 may determine the BPF characteristics based on the detected peak frequency (process P53a).

For example, similar to the first heartbeat detection process, the information processing apparatus 3 may determine BPF characteristics which have the peak frequency at the center frequency and a narrower bandwidth when the peak frequency is higher (a wider bandwidth when the peak frequency is lower) as the BPF characteristics applied to the wireless sensor value.

In addition, the wireless sensor value received by the information processing apparatus 3 in process P51 may be stored in a storage unit 30 in preparation for the above frequency analysis process (P52a). The wireless sensor value stored in the storage unit 30 may be optionally amplified (process P51a) or does not need to be amplified.

The wireless sensor value stored in the storage unit 30 is illustratively read by the processor 31 in process P54a and is applied with a BPF having the BPF characteristics determined in process P53a or P63.

A subsequent feature point detection process (P55) and heart rate calculation process (P56) may be the same as those in the first heartbeat detection process (see, for example, FIG. 20).

As described above, according to the second example of the second heartbeat detection process, the same function and effect as those in the first example are obtained. In addition, the BPF characteristics are controlled based on the wireless sensor value when the inertial sensor value indicates that the influence on detection of a heartbeat component caused by a body motion derived noise component may be regarded to be little.

Accordingly, it is possible to improve a detection accuracy of a heartbeat signal in a band of 0.8 to 1.5 Hz in which the impact due to the body motion derived noise component may be considered as being a little within a band of 0.8 to 4.0 Hz which is an example of the heartbeat appearance band.

(Third heartbeat detection process)

FIG. 30 is a flowchart illustrating an example of the third heartbeat detection process according to one embodiment. The flowchart illustrated in FIG. 30 may be illustratively executed by the processor 31 of the information processing apparatus 3.

In addition, the flowchart illustrated in FIG. 30 may be regarded to correspond to a modified example of the second heartbeat detection process (second example) illustrated in FIG. 21. Hence, in FIG. 30, processes assigned the same reference numerals as those used in FIG. 21 may be regarded to correspond to the same or similar processes as or to the above-described processes in FIG .21.

When determining that the observed person is in a "forward-bent walking" state or a "stooping activity" state, and selecting and determining the third heartbeat detection process, the information processing apparatus 3 may control a detection sensitivity of the wireless sensor 21.

A state where the observed person is in the "forward-bent walking" state or the "stooping activity" state is, for example, a state where the sensor unit 2 is likely to be detached from a human body surface of the observed person. When, for example, the sensor unit 2 is put in a breast pocket of a jacket, and the observed person makes a relative motion, the sensor unit 2 is more likely to be detached from the human body surface of the observed person.

Hence, the sensor unit 2 is likely to move in the direction along the detection axis Z illustrated in FIG. 5B compared to the "resting" state or the "upright walking" state of the observed person.

Hence, the information processing apparatus 3 may set a higher detection sensitivity of the wireless sensor 21 in the third heartbeat detection process than the detection sensitivity of the wireless sensor 21 of the first or second heartbeat detection process.

The detection sensitivity of the wireless sensor 21 can be illustratively changed by controlling (which may be referred to as "correcting" or "adjusting") at least one of transmission power of the wireless sensor 21, a detected signal level of the wireless sensor 21 and a detection threshold of the "feature point" of a heartbeat component.

The transmission power of the wireless sensor 21, the detected signal level of the wireless sensor 21 and the detection threshold of the "feature point" of the heartbeat component maybe regarded as examples of parameters which change the detection sensitivity of the wireless sensor 21 (such parameters may be referred to as "detection sensitivity parameters" for descriptive purposes).

The flowchart in FIG. 30 illustrates as a non-restrictive example an example of correcting detection sensitivity by controlling the transmission power of the wireless sensor 21 which is one of the detection sensitivity parameters in the flowchart illustrated in FIG. 21 (processes P71 and P72).

Illustratively, the information processing apparatus 3 may determine a correction value for the transmission power of the wireless sensor 21 according to the selection and the determination of the third heartbeat detection process, generate a transmission power control signal corresponding to the determined correction value and transmit the transmission power control signal to the sensor unit 2 via the network 4.

The transmission power control signal is received by, for example, the processor 23 of the sensor unit 2 (see FIGS. 2 and 3). The processor 23 controls power of a transmitted radio wave of the OSC 212 by controlling, for example, the MCU 213 of the wireless sensor 21 according to the received transmission power control signal.

Thus, control is performed to adjust the transmission power of the radio wave transmitted from the wireless sensor 21 to higher transmission power than transmission power in the first or second heartbeat detection process. Further, control is performed to adjust the detection sensitivity of the wireless sensor 21 to a detection sensitivity suitable to the "forward-bent walking" state or the "stooping activity" state.

Hence, it is possible to avoid or suppress that a relative distance between a human body surface of the observed person and the sensor unit 2 causes a heartbeat component of the observed person in a wireless sensor value to be detected as an excessively small heartbeat component or an excessively large heartbeat component or makes it difficult to detect the heart component.

In addition, as indicated by a dotted line in FIG. 30, a change target detection sensitivity parameter of the information processing apparatus 3 may be a detected signal level of the wireless sensor 21 or a detection threshold of the "feature point".

For example, the detected signal level of the wireless sensor 21 can be adjusted by varying an amplification factor for amplifying in process P51a the wireless sensor value received in process P51.

For example, the information processing apparatus 3 may perform control to adjust the amplification factor of the received wireless sensor value to a larger amplification factor than the amplification factor in the first or second heartbeat detection process. Consequently, it is possible to adjust the detection sensitivity of the wireless sensor 21 to a detection sensitivity suitable to the "forward-bent walking state" or the "stooping activity" state of the observed person.

Alternatively or additionally, the information processing apparatus 3 may perform control to adjust the threshold value used for the process of detecting the "feature point" in process P55, to a smaller value than the threshold value in the first or second heartbeat detection process. When the threshold value is lowered, the detection sensitivity of the feature point" of a heartbeat component in a wireless sensor value increases. Consequently, it is possible to adjust the detection sensitivity of the wireless sensor 21 to a detection sensitivity suitable to the "forward-bent walking state" or the "stooping activity" state of the observed person.

In addition, above-described detection sensitivity parameter control may be applied to the second example of the second heartbeat detection process illustrated in FIG. 29.

Further, the information processing apparatus 3 may vary the detection sensitivity of the wireless sensor 21 between determination results of the "forward-bent walking" state and the "stooping activity" state (see, for example, FIG. 15).

Illustratively, when the sensor unit 2 is more likely to be detached from the human body surface of the observed person on average in the "stooping activity" state than in the "forward-bent walking" state, the information processing apparatus 3 may perform control to set a higher detection sensitivity for the "stooping activity" than the detection sensitivity for "forward-bent walking".

Further, the information processing apparatus 3 may vary the detection sensitivity of the wireless sensor 21 between the first and second states #1 and #2 (see, for example, FIG. 15) even in the same "stooping" activity state.

Illustratively, in some cases, the sensor unit 2 is more likely to be detached from the human body surface of the observed person in the first state #1 where the observed person stoops to pick up trash or do weeding than in the second state #2 where the observed person mops or vacuums.

In this case, the information processing apparatus 3 may perform control to set a higher detection sensitivity in case where the first state #1 is determined than a detection sensitivity in case where the second state #2 is determined. For example, the information processing apparatus 3 may perform control to set higher transmission power of the wireless sensor 21 in case where the first state #1 is determined than transmission power in case where the second state #2 is determined.

In addition, that the wireless sensor 21 changes a detection sensitivity as described above may also be regarded as an example where the heartbeat detection algorithm is changed.

(Determination example of BPF characteristics)

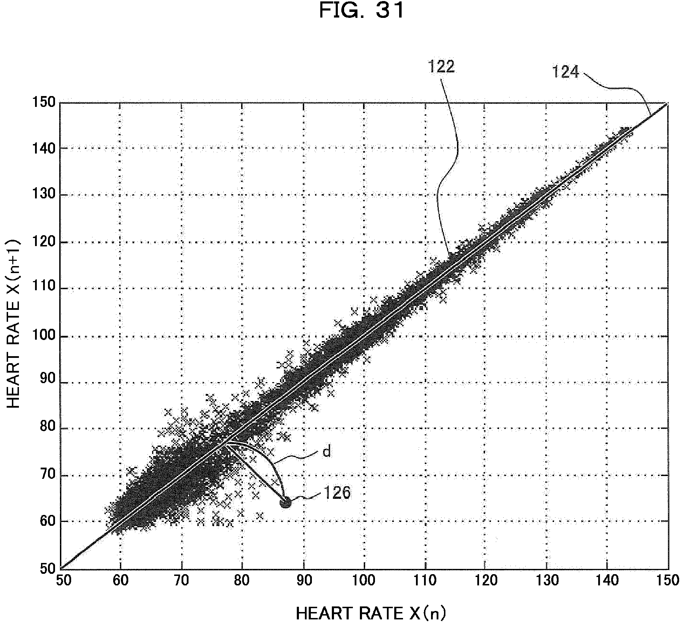

Next, a detailed example of a process (e.g. process P53 in FIG. 20, process P63 in FIGS. 21, 29 and 30 and process P53a in FIG. 29) of determining (or setting) the above-described BPF characteristics will be described with reference to FIGS. 31 to 41.

The determination example of the BPF characteristics described below may be common between the first to third heartbeat detection processes. In this regard, in the following description, a "reference heart rate" corresponds to a heart rate detected based on a wireless sensor value in the first heartbeat detection process, and corresponds to a heart rate estimated based on the inertial sensor value in the first example of the second heartbeat detection process. Further, the "reference heart rate" corresponds to a heart rate estimated based on the inertial sensor value in the second example of the second heartbeat detection process or a heart rate detected based on the wireless sensor value.

For example, in the second example of the second heartbeat detection process, the "reference heart rate" in case where YES is determined in threshold determination process P62a in FIG. 29 corresponds to the heart rate estimated based on the inertial sensor value. By contrast with this, the "reference heart rate" in case where NO is determined in threshold determination process P62a corresponds to the heart rate detected based on the wireless sensor value.

The third heartbeat detection process may be similar to the second heartbeat detection process except that detection sensitivity parameters are controlled. The "reference heart rate" corresponds to a heart rate estimated based on an inertial sensor value or a heart rate detected based on a wireless sensor value.

FIG. 31 illustrates a relation between a heart rate per unit time and a variation of a time length per heartbeat. Reference numeral 122 denotes measured data (x(n), x(n+1)) of the heart rate and a straight line 124 indicates a median value of each measured data 122.

The straight line indicating the median value 124 is illustratively a straight line of each measured data 122 calculated according to a least-squares method, for example, and is assumed to be expressed as ax (n) + bx(n+1) + c = 0. In this regard, coefficients a, b and c are actual numbers.

Here, a length d of a perpendicular line drawn from each measured data 122 (x(n), x(n+1)) to the straight line 124 can be expressed by following equation (9).

$$\mathrm{d} = \frac{|ax(n)+bx(n+1)+c|}{\sqrt{a^2+b^2}} \quad \ldots(9)$$

A measured point having a maximum value of the length d is assumed to be a maximum distance point 126 expressed by a coordinate (x(m), x(m+1)). The "m" represents an positive integer which satisfies m ≤ n.

A straight line which passes through the maximum distance point 126 and a coordinate (a coordinate (120,114) in the example in FIG. 31) corresponding to "-0.1 Hz" in case of heartbeat frequency = 2 Hz represents a lower limit of a bandwidth, for example.

The straight line indicative of an upper limit of the bandwidth corresponds to a straight line which passes through a point (120, 126) and a point having the same width as that of the lower limit of a bandwidth at any of frequencies, for example. A bandwidth in case of heartbeat frequency = 2 Hz may be determined with reference to a maximum value and a minimum value of a next heartbeat at heart rate x (n) = 120 of the measured data 122 in FIG. 31.

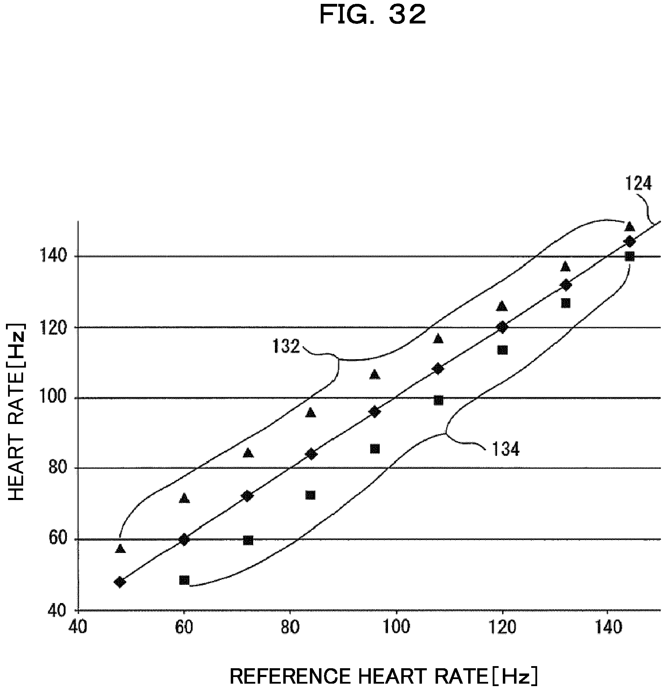

Next, FIG. 32 is a diagram illustrating a setting example of the BPF characteristics. FIG. 32 illustrates an example of an upper limit and a lower limit of a band of the BPF calculated by substituting the reference heart rate in a preset equation.

In FIG. 32, a horizontal axis indicates a reference heart rate [Hz], and a vertical axis indicates a heart rate [Hz] corresponding to an upper limit and a lower limit of a bandwidth. In FIG. 32, the upper limit of the bandwidth is indicated by a band upper limit value 132, and the lower limit of the bandwidth is indicated by a band lower limit value 134. FIG. 32 illustrates the upper limit values and the lower limit values of the bandwidth at some reference heart rate.

The above "preset equation" may be determined by interpolation or extrapolation based on, for example, the maximum distance point 126 and reference coordinates (120, 126) and (120, 114) determined based on the measured data 122 illustrated in FIG. 31.

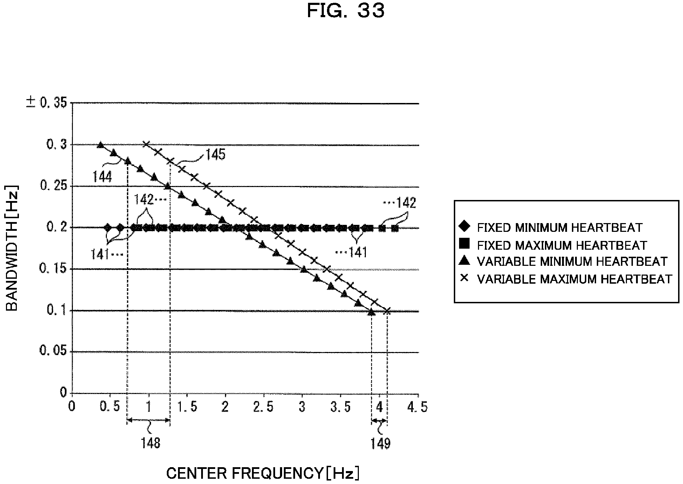

Next, FIG. 33 is a diagram illustrating an example of a passband of the BPF. In FIG. 33, a horizontal axis indicates a center frequency of the BPF, and a vertical axis indicates a bandwidth of the BPF. Further, in FIG. 33, a fixed minimum heartbeat 141 or a variable minimum heartbeat 144 indicates a heart rate corresponding to a lower limit of a band when a bandwidth corresponding to a given center frequency is adopted.

Furthermore, in FIG. 33, a fixed maximum heartbeat 142 or a variable maximum heartbeat 145 indicates a heart rate corresponding to a lower limit of a band when a bandwidth corresponding to a given center frequency is adopted.

The fixed minimum heartbeat 141 and the fixed maximum heartbeat 142 are comparative examples in case where a bandwidth does not change according to a heart rate in comparison with a case where the bandwidth of the BPF is variable in the present embodiment.

According to the variable minimum heartbeat 144 and the variable maximum heartbeat 145, the bandwidth of the BPF corresponds to a bandwidth indicated by reference numeral 148 near heart rate = 1 Hz and corresponds to a bandwidth indicated by reference numeral 149 which is narrower than the bandwidth 148 near heart rate = 4 Hz, for example. Thus, the wider bandwidth 148 is set at a lower heart rate, and the narrower bandwidth 149 is set at a higher heart rate.

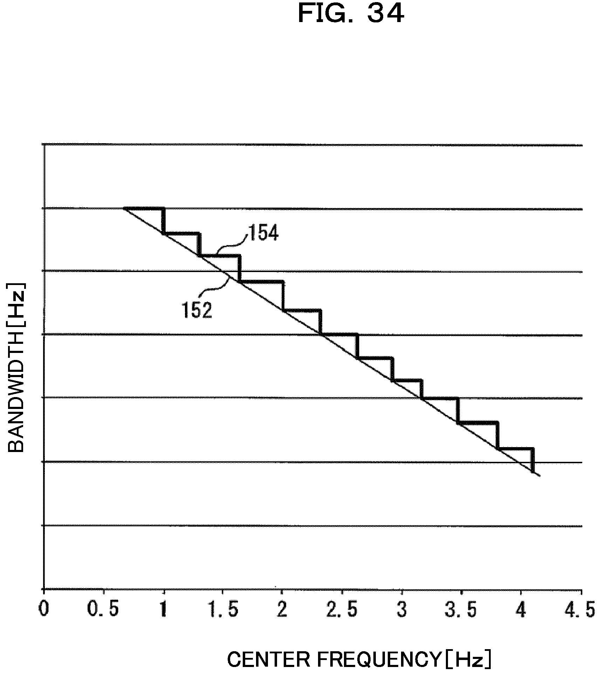

FIG. 34 is a diagram illustrating a setting example of the BPF bandwidth. In FIG. 34, a horizontal axis indicates a center frequency, and a vertical axis indicates a bandwidth. In FIG. 34, a straight line 152 indicates an example of a setting value of a bandwidth with respect to the center frequency. For example, in examples described with reference to FIGS. 31 and 33, the bandwidth 152 may be set to decrease linearly in response to an increase of the center frequency.

A relation between the center frequency and the bandwidth may be enough to have a relation in which the bandwidth becomes wider when the center frequency becomes lower, and does not need to be a relation expressed by a straight line. For example, as indicated by reference numeral 154 in FIG. 34, the bandwidth may be set such that the bandwidth changes stepwise with respect to the center frequency. The relation between the bandwidth and the center frequency may be determined by interpolation and extrapolation using one or more suitable functions of a monotonic increase based on given two or more points determined based on an actual measured value, for example.

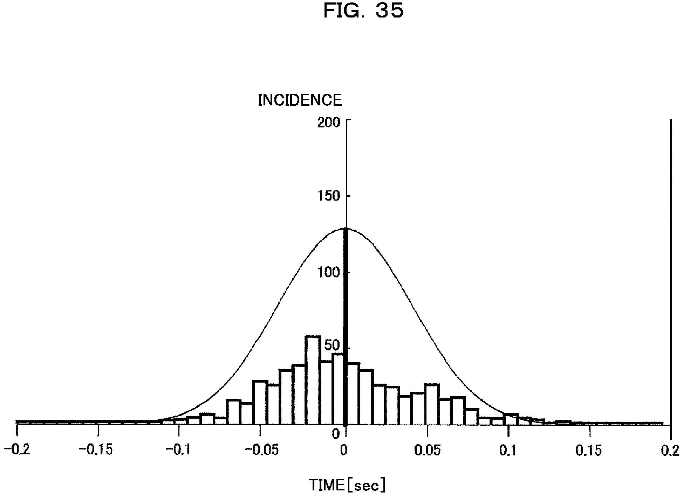

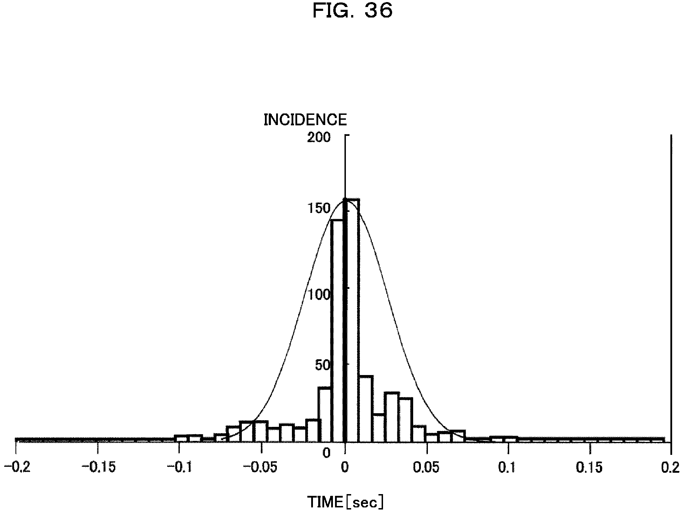

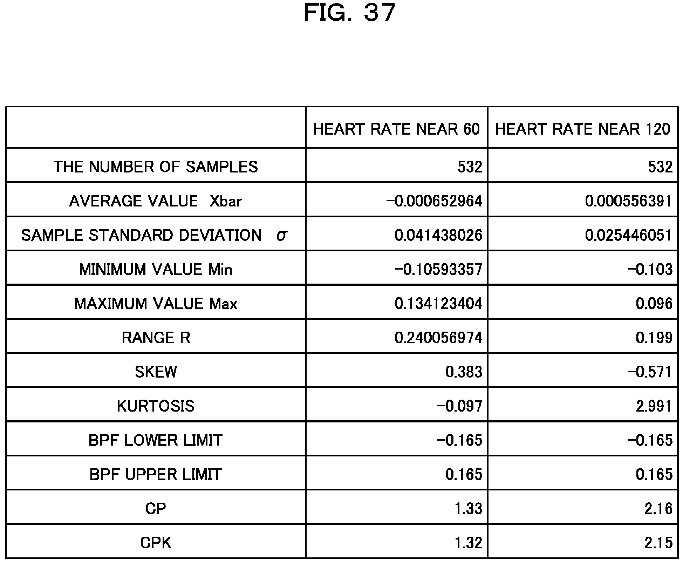

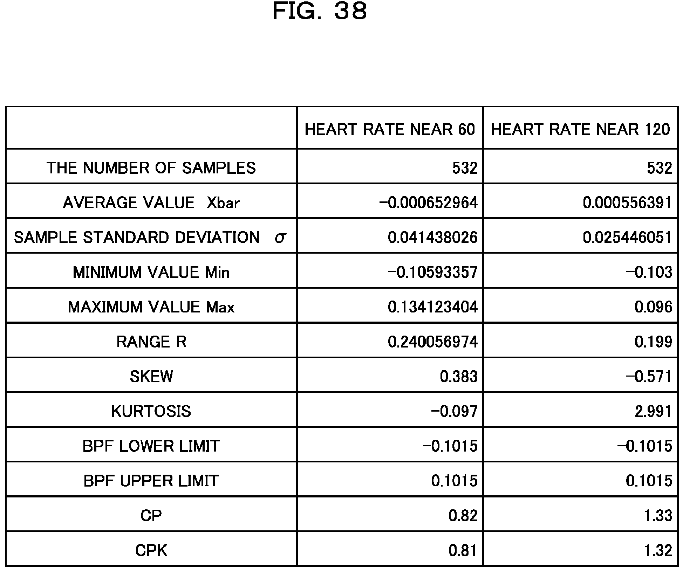

Next, FIGS. 35 to 38 are diagrams illustrating other setting examples of bandwidths. FIGS. 35 and 36 are diagrams illustrating examples of a heart rate distribution, and FIGS. 37 and 38 are diagrams illustrating examples of a heart rate statistical process. In FIGS. 35 and 36, horizontal axes indicate a time indicative of a difference between a heartbeat interval at a given point of time and a next heartbeat interval, and vertical axes indicate the number of measured times.

FIG. 35 illustrates a heart rate distribution whose heart rate is near 55 to 60, and FIG. 36 illustrates a heart rate distribution whose heart rate is near 75 to 80. In comparison between FIGS. 35 and 36, items of measured data concentrate more near the vertical axis at the heart rate near 55 to 60 than at the heart rate near 75 to 80, and the heart rate varies less. The measured data used for a statistical process may be data measured by an electrocardiograph which measures a heartbeat by placing an electrode in contact with a biological object, for example.

FIGS. 37 and 38 illustrate examples of the statistical process of actual measured data at the heart rate near 60 and at the heart rate near 120. In examples of the statistical process illustrated in FIGS. 37 and 38, a probability distribution of 532 items of data is calculated, and the bandwidth of the BPF is calculated such that the measured data is in a range of a certain probability or more.

In the example of the statistical process in FIG. 37, by determining a range of $8\sigma$ of the calculated probability distribution and CP value = 1.33 at the heart rate near 60, the bandwidth is $\pm 0.165$ Hz. Here, $\sigma$ represents a standard deviation, and "CP" represents "Process Capability". The same bandwidth as this bandwidth at the heart rate near 120 is provided in case of CP value = 2.16.

Similarly, in the example of the statistical process in FIG. 38, by determining a range of $8\sigma$ of the calculated probability distribution and CP value = 1.33 at the heart rate near 120, the bandwidth is $\pm 0.1015$ Hz. The same bandwidth as this bandwidth at the heart rate near 60 is provided in case of CP value = 0.82.

The statistical process of an actual measured value is performed as described above, and the bandwidth of the BPF which enables data detection at a given probability or more is set. In this case, the relation between the bandwidth and the center frequency corresponding to the reference heart rate may be linearly determined based on given two points or may be determined by interpolation by performing the above statistical process on the center frequency of a shorter interval.

Alternatively, the relation between the bandwidth and the center frequency may be determined by interpolation and extrapolation by using one or more suitable functions of a monotonic increase based on each point when the given two or more points are determined, or may be set such that the bandwidth changes stepwise with respect to the center frequency.

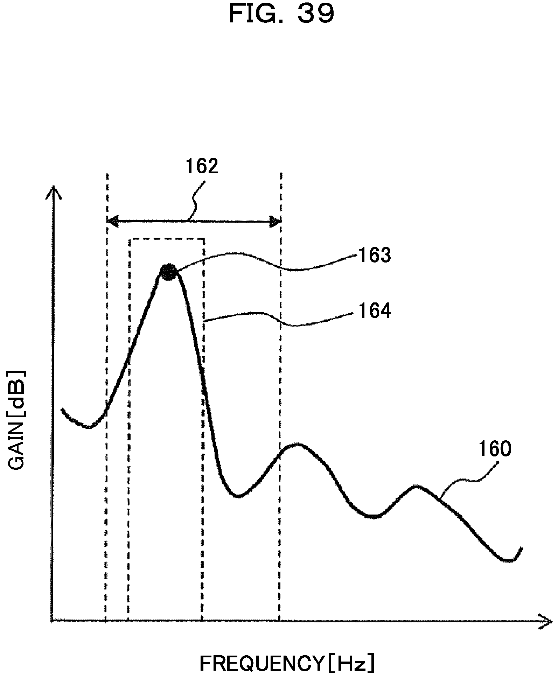

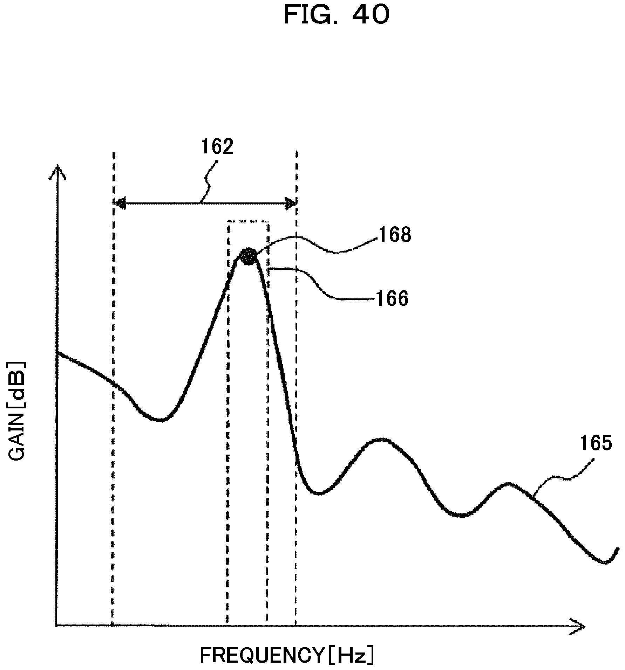

FIGS. 39 and 40 are diagrams illustrating setting examples of a bandwidth. In FIGS. 39 and 40, horizontal axes indicate a frequency [Hz], and vertical axes indicate a gain [dB]. In FIGS. 39 and 40, a heartbeat appearance band 162 is 0.8 to 4.0 Hz, for example.

According to a frequency analysis result (e.g. FFT result) 160 of a wireless sensor value illustrated in FIG. 39, a peak gain indicated by reference numeral 163 exists in the heartbeat appearance band 162. A frequency corresponding to the peak gain 163 is set to the center frequency of the BPF, and a bandwidth indicated by reference numeral 164 is set to the bandwidth of the BPF, for example.

Meanwhile, according to an FFT result 165 illustrated in FIG. 40, a peak gain indicated by reference numeral 168 exists in the heartbeat appearance band 162. The frequency corresponding to the peak gain 168 is set to the center frequency of the BPF.

Here, since the center frequency corresponding to the peak gain 168 is higher than the frequency corresponding to the peak gain 163 in FIG. 39, a bandwidth 166 narrower than the bandwidth 164 in FIG. 39 is set to the bandwidth of the BPF.

In this way, the center frequency of the BPF is set to the frequency corresponding to the reference heart rate, and the bandwidth is adaptively controlled according to a degree of the center frequency. Therefore, it is possible to efficiently remove a noise component which appears in the heartbeat appearance band.

In other words, by using the BPF which has the variable center frequency and variable bandwidth depending on heartbeat characteristics of a biological object, it is possible to efficiently remove an unnecessary signal component in the heartbeat appearance band without depending on a degree of the heart rate. Accordingly, it is possible to improve a detection accuracy of a heartbeat signal in the heartbeat appearance band.

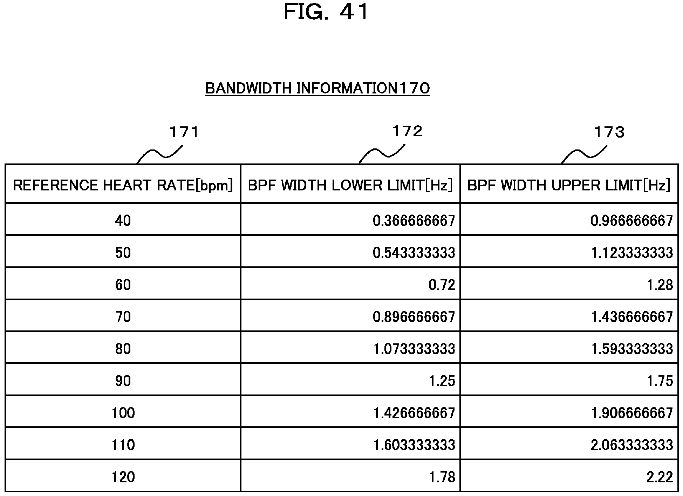

FIG. 41 is a diagram illustrating an example of bandwidth information. Bandwidth information 170 illustrated in FIG. 41 may be stored in the storage unit 30 of the information processing apparatus 3, for example. As a non-restrictive example, the bandwidth information 170 may include reference heart rate information 171, BPF width lower limit information 172 and BPF width upper limit information 173.

The information processing apparatus 3 (e.g. processor 31) can determine and set a band of the BPF associated with the reference heart rate by referring to the bandwidth information 170 in the storage unit 30.

(Others)

In the above-described embodiments including the various examples, a wireless sensor such as a Doppler sensor is used as an example of a heartbeat sensor 21. However, the heartbeat sensor 21 may be a sensor such as an earclip which optically measures a change in a bloodstream, for example.

Even when a bloodstream amount changes due to a body motion, a body motion derived signal component is mixed as a noise component in a sensor detected signal, it is possible to efficiently remove the noise component by controlling the above-described variable BPF characteristics.

In case of the heartbeat sensor 21 which optically measures the change in the flood flow, even when a noise component is mixed in a sensor detected signal on the ground that external light is mixed in reflected light from the bloodstream in outdoor, the noise component can be removed by controlling the above-described variable BPF characteristics.

Further, the heartbeat sensor 21 may be an electrocardiograph or a phonocardiograph which measures a change in a potential of a heart muscle or a change in sound. Even when a body motion derived signal component is mixed as a noise component in a sensor detected signal on the ground that a user's muscle moves due to a body motion, it is possible to efficiently remove the noise component by controlling the above-described variable BPF characteristics.

Further, in the above-described embodiments including the various examples, the heartbeat sensor 21 and the inertial sensor 22 are integrated in the sensor unit 2. However, the heartbeat sensor 21 and the inertial sensor 22 may be provided in separate units as long as the heartbeat sensor 21 and the inertial sensor 22 are attached to the same user. In other words, it is not matter whether the heartbeat sensor 21 and the inertial sensor 22 are integrated or are provided in the separate units as long as a sensing target is the same user.

When the heartbeat sensor 21 and the inertial sensor 22 are integrated in the sensor unit 2, it is possible to omit a labor to individually manage the heartbeat sensor 21 and the inertial sensor 22 or attach the heartbeat sensor 21 and the inertial sensor 22 to the user. Therefore, it is possible to improve user-friendliness and convenience. Further, it is possible to prevent or suppress that one of the heartbeat sensor 21 and the inertial sensor 22 is left without being attached or is lost. Meanwhile, when the heartbeat sensor 21 and the inertial sensor 22 are provided in the separate units, it is possible to individually adjust attachment positions of the respective sensors 21 and 22 for the user. Therefore, it is expected that a degree of freedom in the attachment positions is improved.

**Claims**

1. A sensor information processing apparatus comprising:

   a receiver (34) configured to receive a detected signal of an inertial sensor (22); and
   a processor (31) configured to calculate an extended wavelength defined as a length of a signal waveform corresponding to a locus traced by the detected signal in a time domain per unit time, and to determine an activity state of an observed person observed by the inertial sensor (22) based on the extended wavelength.

2. The sensor information processing apparatus according to claim 1, wherein

   the receiver (34) receives the detected signal of the inertial sensor (22), and a detected signal of a wireless sensor (21) configured to transmit a radio wave toward the observed person, and
   the processor (31) changes an algorithm applied to the detected signal of the wireless sensor to detect a heartbeat component of the observed person from the detected signal of the wireless sensor (21), according to a determination result of the activity state.

3. The sensor information processing apparatus according to claim 1 or 2, wherein

   the inertial sensor (22) includes a first detection axis corresponding to a gravity direction, and
   the processor (31) determines that the activity state of the observed person is in a state of an upright walking, when the extended wavelength on the first detection axis is equal to or more than a first threshold value.

4. The sensor information processing apparatus according to claim 1 or 2, wherein the processor (31) determines that the activity state of the observed person is in a resting state, when the extended wavelength on the first detection axis is equal to or more than a second threshold value which is smaller than the first threshold value

5. The sensor information processing apparatus according to claim 4, wherein the processor (31) detects, in response to the determination indicative of the resting state, a peak frequency having a peak value in a result obtained by performing a frequency analysis on a detected signal of a wireless sensor (21) configured to transmit a radio wave toward the observed person, and detects a heartbeat component of the observed person based on the peak frequency.

6. The sensor information processing apparatus according to claim 1 or 2, wherein

the inertial sensor (22) includes a first detection axis corresponding to a gravity direction, and a second detection axis which is orthogonal to the first detection axis and corresponds to a walking direction of the observed person, and

the processor (31) determines that the activity state of the observed person is in a state of a forward-bent walking, when the extended wavelength on the first detection axis is less than a first threshold value and exceeds a second threshold value smaller than the first threshold value and when an extended wavelength on the second detection axis is less than a third threshold value which is larger than the second threshold value and is smaller than the first threshold value.

7. The sensor information processing apparatus according to claim 6, wherein the processor (31) estimates a heart rate of the observed person based on the detected signal of the inertial sensor (22) in response to a determination indicative of the state of the forward-bent walking,

controls, according to the estimated heart rate, a target frequency band in a detected signal of a wireless sensor (21) configured to transmit a radio wave toward the observed person, the target frequency band being a band in which a heartbeat component of the observed person is to be detected, and

detects the heartbeat component in the target frequency band.

8. The sensor information processing apparatus according to claim 6, wherein

the inertial sensor (22) includes a third detection axis orthogonal to the first and second detection axes, and

the processor (31) performs a subtraction on a difference between extended wavelengths on the second and third detection axes and a difference between extended wavelengths on the first and third detection axes, when the extended wavelength on the second detection axis is equal to or more than the third threshold value, determines that the activity state of the observed person is in a first stooping activity state when a result of the subtraction is equal to or more than a fourth threshold value smaller than the second threshold value, and determines that the activity state of the observed person is in a second stooping activity state where a degree of the stooping is less than the first stooping activity state when the result of the subtraction is less than the fourth threshold value.

9. The sensor information processing apparatus according to claim 8, wherein the processor (31) controls a transmission power of a wireless sensor (21) configured to transmit a radio wave toward the observed person such that the transmission power in a determination of the first stooping activity state is higher than the transmission power in a determination of the second stooping activity state.

10. A sensor unit (2) comprising:

an inertial sensor (22); and

a processor (23) configured to calculate an extended wavelength defined as a length of a signal waveform corresponding to a locus traced by the detected signal of the inertial sensor (22) in a time domain per unit time, and to determine an activity state of an observed person observed by the inertial sensor (22) based on the extended wavelength.

11. The sensor unit (2) according to claim 10, further comprising a wireless sensor (21) configured to transmit a radio wave toward an observed person,

wherein the processor (23) changes an algorithm applied to the detected signal of the wireless sensor to detect a heartbeat component of the observed person from the detected signal of the wireless sensor (21), according to a determination result of the activity state.

12. A sensor information processing program for causing a computer to execute a process comprising:

calculating an extended wavelength defined as a length of a signal waveform corresponding to a locus traced by a detected signal of an inertial sensor (22) in a time domain per unit time; and
determining an activity state of an observed person observed by the inertial sensor (22) based on the extended wavelength.

FIG. 1

1

7 : AIR CONDITIONER

8 : LUMINAIRE

4

NW

3

INFORMATION
PROCESSING
APPARATUS

6

COMMUNICATION
DEVICE

2

SENSOR

# FIG. 2

SENSING
TARGET

2

VITAL SENSOR

INERTIAL
SENSOR 22

211

212 21

OSC

DETECTION
CIRCUIT 214

OP

PROCESSOR

215

216

MCU

POWER SUPPLY
UNIT

213

23

WIRELESS SENSOR

# FIG. 3

2

SENSOR UNIT

23
PROCESSOR

24
MEMORY

25
COMMUNICATION
IF

26

21
WIRELESS
SENSOR

22
INERTIAL
SENSOR

# FIG. 4

3

INFORMATION PROCESSING APPARATUS

31

32

30

34

PROCESSOR

MEMORY

COMMUNICATION IF

36

33

STORAGE DEVICE

PERIPHERAL IF

35

FIG. 5A

2(22)

Y

Z (DETECTION AXES OF
WIRELESS SENSOR)

X

H

W

D

FIG. 5B

Y

X

Z

(DETECTION AXES OF
WIRELESS SENSOR)

2(23)

# FIG. 6

```
        ┌──────────────┐
        │    START     │
        └──────────────┘
                │
                ▼
        ┌──────────────┐
        │RECEIVE INERTIAL│────⌒ P11
        │ SENSOR VALUE │
        └──────────────┘
                │
                ▼
        ┌──────────────┐
        │  CALCULATE   │────⌒ P12
        │  EXTENDED    │
        │  WAVELENGTH  │
        └──────────────┘
                │
                ▼
        ┌─┬──────────┬─┐
        │ │ DETERMINE│ │───⌒ P13
        │ │ ACTIVITY │ │
        │ │  STATE   │ │
        └─┴──────────┴─┘
                │
                ▼
        ┌──────────────┐
        │  DETERMINE   │────⌒ P14
        │  HEARTBEAT   │
        │  DETECTION   │
        │  ALGORITHM   │
        └──────────────┘
                │
                ▼
        ┌─┬──────────┬─┐
        │ │ HEARTBEAT│ │───⌒ P15
        │ │ DETECTION│ │
        │ │ PROCESS  │ │
        └─┴──────────┴─┘
                │
                ▼
   P16⌒┌──────────────┐        ┌──────────────┐
        │   CONTROL    │        │   OUTPUT     │───⌒ P17
        │   SPATIAL    │        │   RESULT     │
        │ ENVIRONMENT  │        └──────────────┘
        └──────────────┘
                │
                ▼
        ┌──────────────┐
        │     END      │
        └──────────────┘
```

31

FIG. 7

SENSOR OUTPUT
(VOLTAGE)[V]

ΔT

A

B

TIME[t]

EXTENDED
WAVELENGTH

# FIG. 8

ORIGINAL WAVEFORM

**FIG. 9A**

DIFFERENTIAL WAVEFORM

**FIG. 9B**

SUM OF DIFFERENTIAL WAVEFORMS FOR ONE SECOND
(EXTENDED WAVEFORM)

**FIG. 9C**

# FIG. 10

FIG. 11

| POSTURE | START OF MEASUREMENT | STAND UPRIGHT (3 MINUTES) | WALK (3 MINUTES) | STAND UPRIGHT (3 MINUTES) | END OF MEASUREMENT |
|---------|---------------------|--------------------------|------------------|--------------------------|--------------------|
| SPEED |  | 0 km/h | 3.6 km/h | 0 km/h |  |

# FIG. 12

EXTENDED WAVEFORM Ly ON DETECTION AXIS Y (PER SEC.)

# FIG. 13

EXTENDED WAVEFORM Ly ON DETECTION AXIS Y (15 SEC.)

# FIG. 14

ACTIVITY STATE
DETERMINATION

ACTIVITY STATE
DETERMINATION

P21

$Ly \geqq THy1?$ — NO

YES

P23

$Ly \leqq THy2?$ — NO

YES

P22 UPRIGHT WALKING
STATE

P24 RESTING
STATE

P25 OTHER STATE

TO PROCESS P14 IN FIG. 6

FIG. 15

TO PROCESS P14 IN FIG. 6

FIG. 16

| POSTURE | START OF MEASUREMENT | STOOPING (3 MINUTES) | FORWARD -BENT WALKING (3 MINUTES) | SQUAT (3 MINUTES) | END OF MEASUREMENT |
|---|---|---|---|---|---|
| SPEED | | 0 km/h | 1.5 km/h | 0 km/h | |

# FIG. 17A

### EXTENDED WAVELENGTH Ly ON DETECTION AXIS Y (PER SEC.)

EXTENDED WAVEFORM PER SEC.

# FIG. 17B

### EXTENDED WAVELENGTH Lz ON DETECTION AXIS Z (PER SEC.)

EXTENDED WAVEFORM PER SEC.

# FIG. 18A

EXTENDED WAVELENGTH Ly ON DETECTION AXIS Y (15 SEC.)

EXTENDED WAVELENGTH FOR 15 SEC.

# FIG. 18B

EXTENDED WAVELENGTH Lz ON DETECTION AXIS Z (15 SEC.)

EXTENDED WAVELENGTH FOR 15 SEC.

# FIG. 19

HEARTBEAT DETECTION ALGORITHM DETERMINATION PROCESS

P141 — REST?
NO
YES

P143 — UPRIGHT WALKING?
NO
YES

P145 — FORWARD-BENT?
NO
YES

P142
FIRST HEARTBEAT DETECTION PROCESS (WIRELESS SENSOR VALUE)

P144
SECOND HEARTBEAT DETECTION PROCESS (WIRELESS SENSOR VALUE + INERTIAL SENSOR VALUE)

P146
THIRD HEARTBEAT DETECTION PROCESS (WIRELESS SENSOR VALUE + INERTIAL SENSOR VALUE + CORRECTION)

P147
OUTPUT ERROR

END

TO PROCESS P16 OR P17 IN FIG. 6

# FIG. 20

## FIRST HEARTBEAT DETECTION PROCESS

```
21 ——⌒              ⌒—— 2
         ┌──────────────┐
         │  WIRELESS    │
         │   SENSOR     │
         └──────────────┘
                │
                ▼
         ┌──────────────┐
P51 ——   │   RECEIVE    │
         │  WIRELESS    │
         │ SENSOR VALUE │
         └──────────────┘
                │
                ▼
P51a---  ┌ ─ ─ ─ ─ ─ ─ ┐
           AMPLIFY
         └ ─ ─ ─ ─ ─ ─ ┘
                │
                ▼
         ┌──────────────┐
P52 ——   │  FREQUENCY   │
         │   ANALYSIS   │
         └──────────────┘
                │
                ▼
         ┌──────────────┐
P53 ——   │ DETERMINE BPF│
         │CHARACTERISTICS│
         └──────────────┘
                │
                ▼
         ┌──────────────┐
P54 ——   │  APPLY BPF   │
         └──────────────┘
                │
                ▼
         ┌──────────────┐
P55 ——   │   DETECT     │
         │ FEATURE POINT│
         └──────────────┘
                │
                ▼
         ┌──────────────┐
P56 ——   │  CALCULATE   │
         │  HEART RATE  │
         └──────────────┘
                │
                ▼
```

TO PROCESS P16 OR P17 IN FIG. 6

# FIG. 21

SECOND HEARTBEAT DETECTION PROCESS
(FIRST EXAMPLE)

```
                                    ┌─2
      ┌─────────────────────────────────────────────────┐
21─┐  │  ┌─────────────┐         ┌─────────────┐  │  ┌─22
      │  │  WIRELESS   │         │  INERTIAL   │  │
      │  │   SENSOR    │         │   SENSOR    │  │
      │  └─────────────┘         └─────────────┘  │
      └─────────────────────────────────────────────────┘
                │                         │
                ▼                         ▼
       ┌─────────────┐           ┌─────────────┐
P51─┐  │   RECEIVE   │           │   RECEIVE   │  ┌─P61
       │  WIRELESS   │           │  INERTIAL   │
       │SENSOR VALUE │           │SENSOR VALUE │
       └─────────────┘           └─────────────┘
                │                         │
                ▼                         ▼
P51a⌐ ┌ ─ ─ ─ ─ ─ ┐            ┌─────────────┐  ┌─P62
        │  AMPLIFY  │            │  ESTIMATE   │
        └ ─ ─ ─ ─ ─ ┘            │ HEART RATE  │
                │                └─────────────┘
                ▼                         │
P54─┐  ┌─────────────┐           ▼          ┌─P63
       │  APPLY BPF  │◄── │ DETERMINE BPF │
       └─────────────┘     │CHARACTERISTICS│
                │          └───────────────┘
                ▼
P55─┐  ┌─────────────┐
       │   DETECT    │
       │FEATURE POINT│
       └─────────────┘
                │
                ▼
P56─┐  ┌─────────────┐
       │  CALCULATE  │
       │ HEART RATE  │
       └─────────────┘
                │
                ▼
```

TO PROCESS P16 OR P17 IN FIG. 6

# FIG. 22

FIRST EXAMPLE OF
HEART RATE ESTIMATION PROCESS

INERTIAL SENSOR VALUE

P62

P621 CALCULATE WALKING CADENCE

P624 ESTIMATE HEART RATE

ESTIMATED HEART RATE

# FIG. 23

$$y = 8E\text{-}05x^3 - 0.0011x^2 - 0.0855x + 61.597$$

# FIG. 24

SECOND EXAMPLE OF
HEART RATE ESTIMATION PROCESS

INERTIAL SENSOR VALUE

P62

CALCULATE
WALKING
CADENCE — P621

CALCULATE
WALKING SPEED — P622

CALCULATE
EXERCISE
INTENSITY — P623

ESTIMATE
HEART RATE — P624

ESTIMATED HEART RATE

# FIG. 25

$$y = 1E\text{-}05x^3 - 0.0014x^2 + 0.0725x - 0.0119$$

WALKING CADENCE[steps/sec]

# FIG. 26

| METs | km/h |
|------|------|
| 1.0 | 0.0 |
| 1.5 | 1.5 |
| 3.0 | 2.5 |
| 3.3 | 3.0 |
| 3.8 | 3.5 |
| 5.0 | 4.0 |
| 6.3 | 4.5 |
| 7.0 | 5.0 |
| 8.0 | 6.6 |
| 9.0 | 8.4 |
| 10.0 | 9.7 |
| 11.0 | 10.8 |
| 15.0 | 14.5 |

# FIG. 27

THIRD EXAMPLE OF
HEART RATE ESTIMATION PROCESS

INERTIAL SENSOR VALUE

P62

CALCULATE
EXTENDED
WAVEFORM — P620
(OR P12 IN FIG. 6)

CALCULATE
EXERCISE
INTENSITY — P623a

ESTIMATE
HEART RATE — P624

ESTIMATED HEART RATE

## FIG. 28A

EXTENDED
WAVELENGTH Lx

$y=1080.2x^2 - 1540.7x - 162.96$

◆ : EXTENDED WAVELENGTH

—— POLYNOMIAL
(EXTENDED WAVELENGTH)

EXERCISE INTENSITY[METs]

## FIG. 28B

EXTENDED
WAVELENGTH Ly

$y=140.95x^2 - 252.5x - 452.54$

◆ : EXTENDED WAVELENGTH

—— POLYNOMIAL
(EXTENDED WAVELENGTH)

EXERCISE INTENSITY[METs]

# FIG. 29

SECOND HEARTBEAT DETECTION PROCESS
(SECOND EXAMPLE)

```
                              ┌──────2
  21 ─┐    ┌─────────────────────────────────────────────┐    ┌─ 22
       └──│   WIRELESS          │      INERTIAL           │────┘
          │   SENSOR            │      SENSOR             │
          └─────────────────────┴─────────────────────────┘
                    │                         │
  P51 ─┐    ┌───────▼────────┐        ┌───────▼────────┐
        └──│   RECEIVE       │        │   RECEIVE      │── P61
           │   WIRELESS      │        │   INERTIAL     │
           │   SENSOR VALUE  │        │   SENSOR VALUE │
           └───────┬────────┘         └───────┬────────┘
                   │                          │
  P51a ─┐    ┌ ─ ─ ▼ ─ ─ ┐                    │
         └ ─ │  AMPLIFY   │                    │
             └ ─ ─ ─ ─ ─ ┘                    │
   WIRELESS SENSOR VALUE (WRITE)              │
```

WIRELESS SENSOR VALUE (WRITE)

30

WIRELESS SENSOR
VALUE (READ)

P52a

| FREQUENCY ANALYSIS |

STORAGE UNIT

ESTIMATE HEART RATE — P62

DETERMINE BPF CHARACTERISTICS

P53a

NO   EXCEED THRESHOLD? — P62a

WIRELESS SENSOR
VALUE (READ)

APPLY BPF

YES

P54a

DETERMINE BPF CHARACTERISTICS — P63

DETECT FEATURE POINT

P55

CALCULATE HEART RATE

P56

TO PROCESS P16 OR 17 IN FIG. 6

# FIG. 30

## THIRD HEARTBEAT DETECTION PROCESS

2

21 — WIRELESS SENSOR

INERTIAL SENSOR — 22

P51 — RECEIVE WIRELESS SENSOR VALUE

RECEIVE INERTIAL SENSOR VALUE — P61

CONTROL TRANSMISSION POWER — P72

P51a — AMPLIFY

ESTIMATE HEART RATE — P62

CALCULATE CORRECTION VALUE — P71

P54 — APPLY BPF

DETERMINE BPF CHARACTERISTICS — P63

P55 — DETECT FEATURE POINT

CORRECT AMPLIFICATION GAIN OR THRESHOLD

P56 — CALCULATE HEART RATE

TO PROCESS P16 OR 17 IN FIG. 6

FIG. 31

FIG. 32

FIG. 33

# FIG. 34

FIG. 35

## FIG. 36

# FIG. 37

|  | HEART RATE NEAR 60 | HEART RATE NEAR 120 |
|---|---|---|
| THE NUMBER OF SAMPLES | 532 | 532 |
| AVERAGE VALUE Xbar | −0.000652964 | 0.000556391 |
| SAMPLE STANDARD DEVIATION $\sigma$ | 0.041438026 | 0.025446051 |
| MINIMUM VALUE Min | −0.10593357 | −0.103 |
| MAXIMUM VALUE Max | 0.134123404 | 0.096 |
| RANGE R | 0.240056974 | 0.199 |
| SKEW | 0.383 | −0.571 |
| KURTOSIS | −0.097 | 2.991 |
| BPF LOWER LIMIT | −0.165 | −0.165 |
| BPF UPPER LIMIT | 0.165 | 0.165 |
| CP | 1.33 | 2.16 |
| CPK | 1.32 | 2.15 |

# FIG. 38

|  | HEART RATE NEAR 60 | HEART RATE NEAR 120 |
|---|---|---|
| THE NUMBER OF SAMPLES | 532 | 532 |
| AVERAGE VALUE Xbar | −0.000652964 | 0.000556391 |
| SAMPLE STANDARD DEVIATION $\sigma$ | 0.041438026 | 0.025446051 |
| MINIMUM VALUE Min | −0.10593357 | −0.103 |
| MAXIMUM VALUE Max | 0.134123404 | 0.096 |
| RANGE R | 0.240056974 | 0.199 |
| SKEW | 0.383 | −0.571 |
| KURTOSIS | −0.097 | 2.991 |
| BPF LOWER LIMIT | −0.1015 | −0.1015 |
| BPF UPPER LIMIT | 0.1015 | 0.1015 |
| CP | 0.82 | 1.33 |
| CPK | 0.81 | 1.32 |

# FIG. 39

# FIG. 40

# FIG. 41

BANDWIDTH INFORMATION170

| 171 | 172 | 173 |
|---|---|---|
| REFERENCE HEART RATE[bpm] | BPF WIDTH LOWER LIMIT[Hz] | BPF WIDTH UPPER LIMIT[Hz] |
| 40 | 0.366666667 | 0.966666667 |
| 50 | 0.543333333 | 1.123333333 |
| 60 | 0.72 | 1.28 |
| 70 | 0.896666667 | 1.436666667 |
| 80 | 1.073333333 | 1.593333333 |
| 90 | 1.25 | 1.75 |
| 100 | 1.426666667 | 1.906666667 |
| 110 | 1.603333333 | 2.063333333 |
| 120 | 1.78 | 2.22 |

**Europäisches Patentamt**
**European Patent Office**
**Office européen des brevets**

# EUROPEAN SEARCH REPORT

Application Number

EP 17 15 3497

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/282717 A1 (MCCOMBIE DEVIN [US] ET AL) 8 October 2015 (2015-10-08)<br>* abstract *<br>* paragraphs [0014], [0021] - [0028], [0063], [0064], [0068], [0084], [0085], [0113] *<br>* figures 5-7 * | 1-12 | INV.<br>A61B5/00<br>A61B5/024<br>A61B5/11 |
| X | US 2014/316305 A1 (VENKATRAMAN SUBRAMANIAM [US] ET AL) 23 October 2014 (2014-10-23)<br>* paragraphs [0097], [0107], [0109], [0168], [0325], [0413] * | 1,10,12 | |
| X | US 2013/041290 A1 (KORDING KONRAD [US] ET AL) 14 February 2013 (2013-02-14)<br>* paragraphs [0017], [0054] - [0063], [0078] - [0081], [0090] - [0092] * | 1,10,12 | |

TECHNICAL FIELDS
SEARCHED        (IPC)

A61B

The present search report has been drawn up for all claims

| Place of search | Date of completion of the search | Examiner |
|---|---|---|
| The Hague | 13 July 2017 | Kowalczyk, Szczepan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...............................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 15 3497

This annex lists the patent family members relating to the patent documents cited in the above-mentioned European search report.
The members are as contained in the European Patent Office EDP file on
The European Patent Office is in no way liable for these particulars which are merely given for the purpose of information.

13-07-2017

| Patent document cited in search report | | Publication date | Patent family member(s) | | Publication date |
|---|---|---|---|---|---|
| US 2015282717 | A1 | 08-10-2015 | EP | 2432380 A1 | 28-03-2012 |
| | | | SG | 176190 A1 | 29-12-2011 |
| | | | SG | 10201403428U A | 26-09-2014 |
| | | | US | 2010298656 A1 | 25-11-2010 |
| | | | US | 2010298657 A1 | 25-11-2010 |
| | | | US | 2010298658 A1 | 25-11-2010 |
| | | | US | 2010298659 A1 | 25-11-2010 |
| | | | US | 2010298660 A1 | 25-11-2010 |
| | | | US | 2010298661 A1 | 25-11-2010 |
| | | | US | 2012190949 A1 | 26-07-2012 |
| | | | US | 2014163393 A1 | 12-06-2014 |
| | | | US | 2015282717 A1 | 08-10-2015 |
| | | | US | 2017150893 A1 | 01-06-2017 |
| | | | WO | 2010135518 A1 | 25-11-2010 |
| US 2014316305 | A1 | 23-10-2014 | US | 2014316305 A1 | 23-10-2014 |
| | | | US | 2017188893 A1 | 06-07-2017 |
| US 2013041290 | A1 | 14-02-2013 | US | 2013041290 A1 | 14-02-2013 |
| | | | WO | 2011133799 A1 | 27-10-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 1115344 A
- JP 2011115459 A
- JP 2006068300 A
- JP 2004081632 A
- JP 2014094043 A

**Non-patent literature cited in the description**

- The METs. National Institute of Health and Nutrition